# EUROPEAN PATENT APPLICATION

(11) **EP 1 586 326 A1**
(43) Date of publication of application: **19.10.2005**
(21) Application number: 04026693.4
(22) Date of filing: 10.11.2004
(51) Int. Cl.: A61K 38/17, A61K 48/00, A61K 35/30, C12N 5/22, A61P 25/28, A61P 9/10

(54) **Pharmaceutical compositions for treating neuronal damage and neurodegenerative diseases**

(30) Priority: 10.11.2003 US 518322; 01.04.2004 JP 2004108669
(71) Applicant: TOUDAI TLO, Ltd., Tokyo 113-0033 (JP)
(72) Inventor: Morimoto, Chikao, Setagaya-ku Tokyo 158-0043 (JP); Sasaki, Takahiro, Shibuya-ku Tokyo 150-0001 (JP)
(74) Representative: Grünecker, Kinkeldey, Stockmair & Schwanhäusser Anwaltssozietät

(57) **Abstract**

Pharmaceutical compositions comprising Nedd9 protein are provided. The pharmaceutical compositions may further comprise FAK protein. These compositions can be used for the treatment of neuronal damages and neurodegenerative diseases. Furthermore, methods for differentiating neuronal cells are provided. The methods comprise introducing a gene encoding Nedd9 protein into neuronal cells and allowing the expression of the gene in the cells.

SEQUENCE LISTING

<110> CENTER FOR ADVANCED SCIENCE AND TECHNOLOGY INCUBATION, LTD.

<120> Pharmaceutical compositions for treating neuronal
      damages and neurodegenerative diseases

<130>

<150> US 60/518,322
<151> 2003-11-10

<150> JP 2004-108669
<151> 2004-4-1

<160> 8

<170> PatentIn Ver. 2.1

<210> 1
<211> 17
<212> DNA
<213> Artificial Sequence

<220>
<223> Description of Artificial Sequence:primer for rat
      Nedd9

<400> 1
aaatgtgggc gaggaat                                                17


<210> 2
<211> 17
<212> DNA
<213> Artificial Sequence

<220>
<223> Description of Artificial Sequence:primer for rat
      Cas-L

<400> 2
agggctcatc tgaccac                                                    17


<210> 3
<211> 18
<212> DNA
<213> Artificial Sequence

<220>
<223> Description of Artificial Sequence:primer for rat
      Nedd9 and Cas-L

<400> 3
tgactggagg gctctttg                                                   18


<210> 4
<211> 20
<212> DNA
<213> Artificial Sequence

<220>
<223> Description of Artificial Sequence:primer for
      beta-actin

<400> 4
ctatgagctg cctgacggtc                                                 20

<210> 5
<211> 833
<212> PRT
<213> Mus musculus

<400> 5
Met Trp Ala Arg Asn Leu Met Ala Arg Ala Leu Tyr Asp Asn Val Pro
1               5                   10                  15

Glu Cys Ala Glu Glu Leu Ala Phe Arg Lys Gly Asp Ile Leu Thr Val
            20                  25                  30

Ile Glu Gln Asn Thr Gly Gly Leu Glu Gly Trp Trp Leu Cys Ser Leu
        35                  40                  45

His Gly Arg Gln Gly Ile Val Pro Gly Asn Arg Val Lys Leu Leu Ile
    50                  55                  60

Gly Pro Val Gln Glu Thr Pro Gly His Glu Gln Pro Thr Pro Gly Pro
65                  70                  75                  80

Met His Gln Thr Phe Gly Gln Gln Lys Leu Tyr Gln Val Pro Asn Ser
                85                  90                  95

Gln Ala Ala Ser Arg Asp Thr Ile Tyr Gln Val Pro Pro Ser Tyr Gln
            100                 105                 110

Asn Gln Gly Ile Tyr Gln Val Pro Thr Gly His Gly Thr Gln Asn Gln
        115                 120                 125

Asp Val Tyr Gln Val Pro Pro Ser Phe Gln Arg Asn Ile Gly Gly Thr
    130                 135                 140

Asn Gly Pro Leu Leu Ser Lys Lys Val Ile Thr Pro Val Arg Thr Gly
145                 150                 155                 160

Leu Gly Tyr Val Tyr Glu Tyr Pro Ser Arg Tyr Gln Lys Asp Val Tyr
                165                 170                 175

Asp Val Pro Pro Ser His Ser Thr Gln Gly Val Tyr Asp Ile Pro Pro
            180                 185                 190

Ser Ser Val Lys Gly Pro Val Phe Ser Val Pro Val Gly Glu Ile Lys
        195                 200                 205

Pro Gln Gly Val Tyr Asp Ile Pro Pro Thr Gln Gly Val Tyr Ala Ile
    210                 215                 220

Pro Pro Ser Ala Cys Arg Asp Glu Ala Gly Leu Arg Glu Lys Glu Tyr
225                 230                 235                 240

Asp Phe Pro Pro Pro Met Lys Gln Asp Gly Lys Pro Asp Thr Arg Pro
                245                 250                 255

Glu Gly Val Tyr Asp Ile Pro Pro Thr Ser Thr Lys Thr Ala Gly Lys
            260                 265                 270

Asp Leu His Ile Lys Phe Pro Cys Asp Ala Pro Gly Gly Val Glu Pro

        275                 280                 285

Met Ala Arg Arg His Gln Ser Phe Ser Leu His His Ala Pro Ser Gln
    290                 295                 300

Leu Gly Gln Ser Gly Asp Thr Gln Ser Asp Ala Tyr Asp Val Pro Arg
305                 310                 315                 320

Gly Val Gln Phe Leu Glu Val Pro Thr Glu Thr Ser Glu Lys Ala Asn
                325                 330                 335

Pro Glu Glu Arg Asp Gly Val Tyr Asp Val Pro Leu His Asn Pro Ala
            340                 345                 350

Asp Ala Lys Gly Ser Arg Asp Val Val Asp Gly Ile Asn Arg Leu Ser
        355                 360                 365

Phe Ser Ser Thr Gly Ser Thr Arg Ser Asn Met Ser Thr Ser Ser Thr
    370                 375                 380

Ser Ser Lys Glu Ser Ser Leu Ser Ala Ser Pro Ser Gln Asp Lys Arg
385                 390                 395                 400

Leu Arg Leu Asp Pro Asp Thr Ala Ile Glu Lys Leu Tyr Arg Leu Gln
                405                 410                 415

Gln Thr Leu Glu Met Gly Val Cys Ser Leu Met Ser Leu Val Thr Thr
            420                 425                 430

Asp Trp Arg Cys Tyr Gly Tyr Met Glu Arg His Ile Asn Glu Ile Arg
        435                 440                 445

Thr Ala Val Asp Lys Val Glu Leu Phe Leu Arg Glu Tyr Leu His Phe
    450                 455                 460

Ala Lys Gly Ala Leu Ala Asn Ala Ser Cys Leu Pro Glu Leu Val Leu
465                 470                 475                 480

His Asn Lys Met Ser Arg Glu Leu Gln Arg Val Glu Asp Ser His Gln
                485                 490                 495

Ile Leu Ser Gln Thr Ser His Glu Leu Asp Glu Cys Ser Trp Ser Leu
            500                 505                 510

Asn Ile Leu Ala Ile Asn Lys Pro Gln Asn Lys Cys Asp Gly Leu Asp
        515                 520                 525

Arg Phe Val Met Val Ala Lys Thr Val Pro Asp Asp Ala Lys Gln Leu
    530                 535                 540

Thr Thr Thr Ile Ser Thr Tyr Ala Glu Thr Leu Phe Arg Ala Asp Pro
545                 550                 555                 560

Ala Asn Ser His Leu Lys Asn Gly Pro Asn Ser Ile Met Asn Ser Ser
                565                 570                 575

Glu Tyr Thr His Pro Gly Ser Gln Met Gln Pro Leu His Pro Gly Asp
            580                 585                 590

Tyr Lys Ala Gln Val His Ser Lys Pro Leu Pro Pro Ser Leu Ser Lys
        595                 600                 605

Asp Gln Pro Pro Asp Cys Gly Ser Ser Asp Gly Ser Glu Arg Ser Trp
    610                 615                 620

Met Asp Asp Tyr Asp Tyr Val His Leu Gln Gly Lys Glu Glu Phe Glu
625                 630                 635                 640

Arg Gln Gln Lys Glu Leu Leu Glu Lys Ala Asn Ile Met Lys Gln Ser
                645                 650                 655

Lys Ala Gln Leu Glu His His Gln Leu Ser Gln Phe Gln Leu Leu Glu
            660                 665                 670

Gln Glu Ile Thr Lys Pro Val Glu Asn Asp Ile Ser Lys Trp Lys Pro
        675                 680                 685

Ser Gln Ser Leu Pro Thr Thr Asn Asn Ser Val Gly Ala Gln Asp Arg
    690                 695                 700

Gln Leu Leu Cys Phe Tyr Tyr Asp Gln Cys Glu Thr His Phe Ile Ser
705                 710                 715                 720

Leu Leu Asn Ala Ile Asp Ala Leu Phe Ser Cys Val Thr Pro Ala Gln
                725                 730                 735

Pro Pro Arg Ile Phe Val Ala His Ser Lys Phe Val Ile Leu Ser Ala

            740                 745                 750

His Lys Leu Val Phe Ile Gly Asp Thr Leu Thr Arg Gln Val Ala Ala
        755                 760                 765

Gln Asp Ile Arg Asn Lys Val Arg Asn Ser Ser Asn Gln Leu Cys Glu
    770                 775                 780

Gln Leu Lys Thr Ile Val Met Ala Thr Lys Met Ala Ala Leu His Tyr
785                 790                 795                 800

Pro Ser Thr Thr Ala Leu Gln Glu Met Val His Gln Val Thr Asp Leu
                805                 810                 815

Ser Arg Asn Ala Gln Leu Phe Lys Arg Ser Leu Leu Glu Met Ala Thr
            820                 825                 830

Phe

<210> 6
<211> 833
<212> PRT
<213> Rattus norvegicus

<400> 6
Met Trp Ala Arg Asn Leu Met Ala Arg Ala Leu Tyr Asp Asn Val Pro
1               5                   10                  15

Glu Cys Ala Glu Glu Leu Ala Phe Arg Lys Gly Asp Ile Leu Thr Val
            20                  25                  30
Ile Glu Gln Asn Thr Gly Gly Leu Glu Gly Trp Trp Leu Cys Ser Leu
        35                  40                  45
His Gly Arg Gln Gly Ile Val Pro Gly Asn Arg Val Lys Leu Leu Ile
    50                  55                  60
Gly Pro Val Gln Glu Pro Ser Ser His Glu Gln Pro Thr Pro Gly Pro
65                  70                  75                  80
Met His Gln Asn Phe Gly Gln Gln Lys Leu Tyr Gln Val Pro Asn Ser
                85                  90                  95
Gln Ala Ala Pro Arg Asp Thr Ile Tyr Gln Val Pro Pro Ser Tyr Gln
            100                 105                 110
Asn Gln Gly Ile Tyr Gln Val Pro Thr Gly His Gly Thr Pro Glu Gln
        115                 120                 125
Asp Val Tyr Gln Val Pro Pro Ser Val Gln Arg Asn Ile Gly Ser Thr
    130                 135                 140
Asn Gly Pro His Leu Gly Lys Lys Val Ile Thr Pro Val Arg Val Gly
145                 150                 155                 160
His Gly Tyr Val Tyr Glu His Pro Ser Arg Tyr Gln Lys Asp Val Tyr
                165                 170                 175

Asp Val Pro Pro Ser His Thr Thr Gln Gly Val Tyr Asp Ile Pro Pro
            180                 185                 190

Ser Ser Val Lys Gly Pro Val Phe Ser Val Pro Val Gly Glu Leu Lys
        195                 200                 205

Pro Gln Gly Val Tyr Asp Ile Pro Pro Thr Gln Gly Val Tyr Ala Ile
    210                 215                 220

Pro Pro Ser Ala Cys Arg Asp Glu Ala Gly Leu Arg Glu Lys Glu Tyr
225                 230                 235                 240

Asp Phe Pro Pro Pro Met Lys Gln Asp Gly Lys Leu Asp Thr Arg Pro
                245                 250                 255

Glu Gly Val Tyr Asp Ile Pro Pro Thr Ser Thr Lys Thr Val Gly Lys
            260                 265                 270

Asp Leu His Thr Lys Phe Pro Cys Asp Ala Pro Gly Gly Ala Glu Pro
        275                 280                 285

Met Ala Arg Arg His Gln Ser Leu Ser Leu His His Ala Pro Ser Gln
    290                 295                 300

Val Gly Pro Ser Gly Asp Thr Gln Ser Asp Ala Tyr Asp Val Pro Arg
305                 310                 315                 320

Gly Val Gln Phe Leu Glu Val Pro Thr Glu Ser Ser Glu Lys Ala Asn

                325                 330                 335

Leu Glu Glu Arg Asp Gly Val Tyr Asp Val Pro Leu His Asn Pro Ala
            340                 345                 350

Asp Ala Lys Gly Ser Arg Asp Val Val Asp Gly Ile Asn Arg Leu Ser
        355                 360                 365

Phe Ser Ser Thr Gly Ser Thr Arg Ser Asn Met Ser Thr Ser Ser Thr
    370                 375                 380

Ser Ser Lys Glu Ser Ser Leu Ser Ala Ser Pro Ser Gln Asp Lys Arg
385                 390                 395                 400

Leu Leu Leu Asp Pro Asp Thr Ala Ile Gly Lys Leu Tyr Trp Leu Gln
                405                 410                 415

Gln Thr Leu Glu Thr Gly Ile Cys Ser Leu Met Ser Leu Val Thr Ile
            420                 425                 430

Asp Trp Arg Ser Tyr Gly Tyr Met Glu Arg His Ile Asn Glu Ile Arg
        435                 440                 445

Thr Ala Val Asp Lys Val Glu Leu Phe Leu Arg Glu Tyr Leu His Phe
    450                 455                 460

Ala Arg Gly Ala Leu Ala Asn Ala Ser Cys Leu Pro Glu Pro Val Leu
465                 470                 475                 480

Leu Asn Lys Met Asn Arg Glu Leu Gln Arg Val Asp Asp Ser His Gln
                485                 490                 495

Ile Leu Ser Gln Thr Ser His Asp Leu Asn Glu Cys Ser Trp Ser Leu
            500                 505                 510

Asn Val Leu Ala Ile Asn Lys Pro Gln Asn Lys Cys Asp Asp Leu Asp
        515                 520                 525

Arg Phe Val Met Ala Val Lys Thr Val Pro Asp Asp Ala Lys Gln Leu
    530                 535                 540

Thr Thr Thr Ile Ser Thr Tyr Ala Glu Thr Leu Phe Arg Val Asp Pro
545                 550                 555                 560

Gly Asn Ser His Leu Lys Asn Gly Pro Asp Ser Ile Thr Asn Ser Ile
                565                 570                 575

Glu Tyr Thr His Thr Gly Ser Gln Met Gln Pro Leu Arg Pro Gly Asp
            580                 585                 590

Tyr Lys Gly Gln Val His Asn Lys Pro Leu Pro Pro Thr Leu Ser Lys
        595                 600                 605

Glu Gln Pro Pro Asp Cys Ser Ser Ser Asp Gly Ser Glu Arg Ser Trp
    610                 615                 620

Met Asp Asp Tyr Asp Tyr Val His Leu Gln Gly Lys Glu Glu Phe Glu
625                 630                 635                 640

Arg Gln Gln Lys Glu Leu Leu Glu Lys Glu Asn Ile Met Lys Gln Ser
                645                 650                 655

Lys Ala Gln Leu Glu His His Gln Leu Ser Gln Phe Gln Leu Leu Glu
            660                 665                 670

Gln Glu Ile Thr Lys Pro Val Glu Asn Asp Ile Ser Lys Trp Lys Pro
        675                 680                 685

Ser Gln Ser Leu Pro Thr Thr Asn Asn Ser Val Gly Ala Gln Asp Arg
    690                 695                 700

Gln Leu Leu Cys Phe Tyr Tyr Asp Gln Cys Glu Thr His Phe Ile Ser
705                 710                 715                 720

Leu Leu Asn Ala Ile Asp Ala Leu Phe Ser Cys Val Ser Ser Ala Gln
                725                 730                 735

Pro Pro Arg Ile Phe Val Ala His Ser Lys Phe Val Ile Leu Ser Ala
            740                 745                 750

His Lys Leu Val Phe Ile Gly Asp Thr Leu Thr Arg Gln Val Ala Ala
        755                 760                 765

Gln Asp Ile Arg Asn Lys Val Arg Asn Ser Ser Asn Gln Leu Cys Glu
    770                 775                 780

Gln Leu Lys Thr Ile Val Met Ala Thr Lys Met Ala Ala Leu His Tyr

785                 790                 795                 800

Pro Ser Thr Thr Ala Leu Gln Glu Met Val His Gln Val Thr Asp Leu
                805                 810                 815

Ser Arg Asn Ala Gln Leu Phe Lys Arg Ser Leu Leu Glu Met Ala Thr
            820                 825                 830

Phe

<210>  7
<211>  833
<212>  PRT
<213>  Rattus norvegicus

<400>  7
Met Lys Tyr Lys Asn Leu Met Ala Arg Ala Leu Tyr Asp Asn Val Pro
  1               5                  10                  15

Glu Cys Ala Glu Glu Leu Ala Phe Arg Lys Gly Asp Ile Leu Thr Val
             20                  25                  30

Ile Glu Gln Asn Thr Gly Gly Leu Glu Gly Trp Trp Leu Cys Ser Leu
         35                  40                  45

His Gly Arg Gln Gly Ile Val Pro Gly Asn Arg Val Lys Leu Leu Ile
     50                  55                  60

Gly Pro Val Gln Glu Pro Ser Ser His Glu Gln Pro Thr Pro Gly Pro
65                  70                  75                  80

Met His Gln Asn Phe Gly Gln Gln Lys Leu Tyr Gln Val Pro Asn Ser
                85                  90                  95

Gln Ala Ala Pro Arg Asp Thr Ile Tyr Gln Val Pro Pro Ser Tyr Gln
            100                 105                 110

Asn Gln Gly Ile Tyr Gln Val Pro Thr Gly His Gly Thr Pro Glu Gln
        115                 120                 125

Asp Val Tyr Gln Val Pro Pro Ser Val Gln Arg Asn Ile Gly Ser Thr
    130                 135                 140

Asn Gly Pro His Leu Gly Lys Lys Val Ile Thr Pro Val Arg Val Gly
145                 150                 155                 160

His Gly Tyr Val Tyr Glu His Pro Ser Arg Tyr Gln Lys Asp Val Tyr
                165                 170                 175

Asp Val Pro Pro Ser His Thr Thr Gln Gly Val Tyr Asp Ile Pro Pro
            180                 185                 190

Ser Ser Val Lys Gly Pro Val Phe Ser Val Pro Val Gly Glu Leu Lys
        195                 200                 205

Pro Gln Gly Val Tyr Asp Ile Pro Pro Thr Gln Gly Val Tyr Ala Ile
    210                 215                 220

Pro Pro Ser Ala Cys Arg Asp Glu Ala Gly Leu Arg Glu Lys Glu Tyr
225                 230                 235                 240

Asp Phe Pro Pro Pro Met Lys Gln Asp Gly Lys Leu Asp Thr Arg Pro
                245                 250                 255

Glu Gly Val Tyr Asp Ile Pro Pro Thr Ser Thr Lys Thr Val Gly Lys
            260                 265                 270

Asp Leu His Thr Lys Phe Pro Cys Asp Ala Pro Gly Gly Ala Glu Pro
        275                 280                 285

Met Ala Arg Arg His Gln Ser Leu Ser Leu His His Ala Pro Ser Gln
    290                 295                 300

Val Gly Pro Ser Gly Asp Thr Gln Ser Asp Ala Tyr Asp Val Pro Arg
305                 310                 315                 320

Gly Val Gln Phe Leu Glu Val Pro Thr Glu Ser Ser Glu Lys Ala Asn
                325                 330                 335

Leu Glu Glu Arg Asp Gly Val Tyr Asp Val Pro Leu His Asn Pro Ala
            340                 345                 350

Asp Ala Lys Gly Ser Arg Asp Val Val Asp Gly Ile Asn Arg Leu Ser
        355                 360                 365

Phe Ser Ser Thr Gly Ser Thr Arg Ser Asn Met Ser Thr Ser Ser Thr

    370                 375                 380

Ser Ser Lys Glu Ser Ser Leu Ser Ala Ser Pro Ser Gln Asp Lys Arg
385                 390                 395                 400

Leu Leu Leu Asp Pro Asp Thr Ala Ile Gly Lys Leu Tyr Trp Leu Gln
                405                 410                 415

Gln Thr Leu Glu Thr Gly Ile Cys Ser Leu Met Ser Leu Val Thr Ile
            420                 425                 430

Asp Trp Arg Ser Tyr Gly Tyr Met Glu Arg His Ile Asn Glu Ile Arg
        435                 440                 445

Thr Ala Val Asp Lys Val Glu Leu Phe Leu Arg Glu Tyr Leu His Phe
    450                 455                 460

Ala Arg Gly Ala Leu Ala Asn Ala Ser Cys Leu Pro Glu Pro Val Leu
465                 470                 475                 480

Leu Asn Lys Met Asn Arg Glu Leu Gln Arg Val Asp Asp Ser His Gln
                485                 490                 495

Ile Leu Ser Gln Thr Ser His Asp Leu Asn Glu Cys Ser Trp Ser Leu
            500                 505                 510

Asn Val Leu Ala Ile Asn Lys Pro Gln Asn Lys Cys Asp Asp Leu Asp
        515                 520                 525

Arg Phe Val Met Val Ala Lys Thr Val Pro Asp Asp Ala Lys Gln Leu
    530                 535                 540

Thr Thr Thr Ile Ser Thr Tyr Ala Glu Thr Leu Phe Arg Val Asp Pro
545                 550                 555                 560

Gly Asn Ser His Leu Lys Asn Gly Pro Asp Ser Ile Thr Asn Ser Ile
                565                 570                 575

Glu Tyr Thr His Thr Gly Ser Gln Met Gln Pro Leu Arg Pro Gly Asp
            580                 585                 590

Tyr Lys Gly Gln Val His Asn Lys Pro Leu Pro Pro Thr Leu Ser Lys
        595                 600                 605

Glu Gln Pro Pro Asp Cys Ser Ser Ser Asp Gly Ser Glu Arg Ser Trp
    610                 615                 620

Met Asp Asp Tyr Asp Tyr Val His Leu Gln Gly Lys Glu Glu Phe Glu
625                 630                 635                 640

Arg Gln Gln Lys Glu Leu Leu Glu Lys Glu Asn Ile Met Lys Gln Ser
                645                 650                 655

Lys Ala Gln Leu Glu His His Gln Leu Ser Gln Phe Gln Leu Leu Glu
            660                 665                 670

Gln Glu Ile Thr Lys Pro Val Glu Asn Asp Ile Ser Lys Trp Lys Pro
        675                 680                 685

Ser Gln Ser Leu Pro Thr Thr Asn Asn Ser Val Gly Ala Gln Asp Arg
    690                 695                 700

Gln Leu Leu Cys Phe Tyr Tyr Asp Gln Cys Glu Thr His Phe Ile Ser
705                 710                 715                 720

Leu Leu Asn Ala Ile Asp Ala Leu Phe Ser Cys Val Ser Ser Ala Gln
                725                 730                 735

Pro Pro Arg Ile Phe Val Ala His Ser Lys Phe Val Ile Leu Ser Ala
            740                 745                 750

His Lys Leu Val Phe Ile Gly Asp Thr Leu Thr Arg Gln Val Ala Ala
        755                 760                 765

Gln Asp Ile Arg Asn Lys Val Arg Asn Ser Ser Asn Gln Leu Cys Glu
    770                 775                 780

Gln Leu Lys Thr Ile Val Met Ala Thr Lys Met Ala Ala Leu His Tyr
785                 790                 795                 800

Pro Ser Thr Thr Ala Leu Gln Glu Met Val His Gln Val Thr Asp Leu
                805                 810                 815

Ser Arg Asn Ala Gln Leu Phe Lys Arg Ser Leu Leu Glu Met Ala Thr
            820                 825                 830

Phe

<210> 8

<211> 832

<212> PRT

<213> Mus musculus

<400> 8

Met Lys Tyr Lys Asn Leu Met Ala Arg Ala Leu Tyr Asp Asn Val Pro
1               5                   10                  15

Glu Cys Ala Glu Glu Leu Ala Phe Arg Lys Gly Asp Ile Leu Thr Val
            20                  25                  30

Ile Glu Gln Asn Thr Gly Gly Leu Glu Gly Trp Trp Leu Arg Ser Leu
        35                  40                  45

His Gly Arg Gln Gly Ile Val Pro Gly Asn Arg Val Lys Leu Leu Ile
    50                  55                  60

Gly Pro Val Gln Glu Thr Pro Gly His Glu Gln Pro Thr Pro Gly Pro
65                  70                  75                  80

Met His Gln Thr Phe Gly Gln Gln Lys Leu Tyr Gln Val Pro Asn Ser
                85                  90                  95

Gln Ala Ala Ser Arg Asp Thr Ile Tyr Gln Val Pro Pro Ser Tyr Gln
            100                 105                 110

Asn Gln Gly Ile Tyr Gln Val Pro Thr Gly His Gly Thr Pro Glu Gln
        115                 120                 125

Asp Val Tyr Gln Val Pro Pro Ser Val Gln Arg Asn Ile Gly Gly Thr
    130                 135                 140

Asn Gly Pro Leu Leu Ser Lys Lys Val Ile Thr Pro Val Arg Thr Gly
145                 150                 155                 160

His Gly Tyr Val Tyr Glu Tyr Pro Ser Arg Tyr Gln Lys Asp Val Tyr
                165                 170                 175

Asp Val Pro Pro Ser His Ser Thr Gln Gly Val Tyr Asp Ile Pro Pro
            180                 185                 190

Ser Ser Val Lys Gly Pro Val Phe Ser Val Pro Val Gly Glu Ile Lys
        195                 200                 205

Pro Gln Gly Val Tyr Asp Ile Pro Pro Thr Gln Gly Val Tyr Ala Ile
    210                 215                 220

Pro Pro Ser Ala Cys Arg Asp Glu Ala Gly Leu Arg Glu Lys Glu Tyr
225                 230                 235                 240

Asp Phe Pro Pro Pro Met Lys Gln Asp Gly Lys Pro Asp Thr Arg Pro
                245                 250                 255

Glu Gly Val Tyr Asp Ile Pro Pro Thr Ser Thr Lys Thr Ala Gly Lys
            260                 265                 270

Asp Leu His Ile Lys Phe Pro Cys Asp Ala Pro Gly Gly Val Glu Pro
        275                 280                 285

Met Ala Arg Arg His Gln Ser Phe Ser Leu His His Ala Pro Ser Gln
    290                 295                 300

Leu Gly Gln Ser Gly Asp Thr Gln Ser Asp Ala Tyr Asp Val Pro Arg
305                 310                 315                 320

Gly Val Gln Phe Leu Glu Val Pro Thr Glu Thr Ser Glu Lys Ala Asn
                325                 330                 335

Pro Glu Glu Arg Asp Gly Val Tyr Asp Val Pro Leu His Asn Pro Ala
            340                 345                 350

Asp Ala Lys Gly Ser Arg Asp Val Val Asp Gly Ile Asn Arg Leu Ser
        355                 360                 365

Phe Ser Ser Thr Gly Ser Thr Arg Ser Asn Met Ser Thr Ser Ser Thr
    370                 375                 380

Ser Ser Lys Glu Ser Ser Leu Ser Ala Ser Pro Ser Gln Asp Lys Arg
385                 390                 395                 400

Leu Arg Leu Asp Pro Asp Thr Ala Ile Glu Lys Leu Tyr Arg Leu Gln
                405                 410                 415

Gln Thr Leu Glu Met Gly Val Cys Ser Leu Met Ser Leu Val Thr Thr

            420                 425                 430

Asp Trp Arg Cys Tyr Gly Tyr Met Glu Arg His Ile Asn Glu Ile Arg
        435                 440                 445

Thr Ala Val Asp Lys Val Glu Leu Phe Leu Arg Glu Tyr Leu His Phe
    450                 455                 460

Ala Lys Gly Ala Leu Ala Asn Ala Ser Cys Leu Pro Glu Leu Val Leu
465                 470                 475                 480

His Asn Lys Met Lys Arg Glu Leu Gln Arg Val Glu Asp Ser His Gln
                485                 490                 495

Ile Leu Ser Gln Thr Ser His Asp Leu Asn Glu Cys Ser Trp Ser Leu
            500                 505                 510

Asn Ile Leu Ala Ile Asn Lys Pro Gln Asn Lys Cys Asp Asp Leu Asp
        515                 520                 525

Arg Phe Val Met Val Ala Lys Thr Val Pro Asp Asp Ala Lys Gln Leu
    530                 535                 540

Thr Thr Thr Ile Ser Thr Tyr Ala Glu Thr Leu Phe Arg Ala Asp Pro
545                 550                 555                 560

Ala Asn Ser His Leu Lys Asn Gly Pro Asn Ser Ile Met Asn Ser Ser
                565                 570                 575

Glu Tyr Thr His Pro Gly Ser Gln Met Gln Pro Leu His Pro Gly Asp
            580                 585                 590

Tyr Lys Ala Gln Val His Ser Lys Pro Leu Pro Pro Ser Leu Ser Lys
        595                 600                 605

Asp Gln Pro Pro Asp Cys Gly Ser Ser Asp Gly Ser Glu Arg Ser Trp
    610                 615                 620

Met Asp Asp Tyr Asp Tyr Val His Leu Gln Gly Lys Glu Glu Phe Glu
625                 630                 635                 640

Arg Gln Gln Lys Glu Leu Leu Glu Lys Glu Asn Ile Met Lys Gln Ser
                645                 650                 655

Lys Ala Gln Leu Glu His His Gln Leu Ser Gln Phe Gln Leu Leu Glu
            660                 665                 670

Gln Glu Ile Thr Lys Pro Val Glu Asn Asp Ile Ser Lys Trp Lys Pro
        675                 680                 685

Ser Gln Ser Leu Pro Thr Thr Asn Asn Ser Val Gly Ala Gln Asp Arg
    690                 695                 700

Gln Leu Leu Cys Phe Tyr Tyr Asp Gln Cys Glu Thr His Phe Ile Ser
705                 710                 715                 720

Leu Leu Asn Ala Ile Asp Ala Leu Phe Ser Cys Val Ser Ser Ala Gln
                725                 730                 735

Pro Pro Arg Ile Phe Val Ala His Ser Lys Phe Val Ile Leu Ser Ala
            740                 745                 750
His Lys Leu Val Phe Ile Gly Asp Thr Leu Thr Arg Gln Val Ala Ala
        755                 760                 765
Gln Asp Ile Arg Asn Lys Val Arg Asn Ser Ser Asn Gln Leu Cys Glu
    770                 775                 780
Gln Leu Lys Thr Ile Val Met Ala Thr Lys Met Ala Ala Leu His Tyr
785                 790                 795                 800
Pro Ser Thr Thr Ala Leu Gln Glu Met Val His Gln Val Thr Asp Leu
                805                 810                 815
Ser Arg Asn Ala Gln Leu Phe Lys Arg Ser Leu Leu Glu Met Ala Thr
            820                 825                 830

SEQUENCE LISTING

<110> TOUDAI TLO, LTD.

<120> Pharmaceutical compositions for treating neuronal
      damages and neurodegenerative diseases

<130> EP32994HVpau

<140> EP 04026693.4
<141> 2004-11-10

<150> US 60/518,322
<151> 2003-11-10

<150> JP 2004-108669
<151> 2004-04-01

<160> 8

<170> PatentIn Ver. 2.1

<210> 1
<211> 17
<212> DNA
<213> Artificial Sequence

<220>
<223> Description of Artificial Sequence:primer for rat
      Nedd9

<400> 1
aaatgtgggc gaggaat                                                17


<210> 2
<211> 17
<212> DNA
<213> Artificial Sequence

<220>
<223> Description of Artificial Sequence:primer for rat
      Cas-L

<400> 2
agggctcatc tgaccac                                                17


<210> 3
<211> 18
<212> DNA
<213> Artificial Sequence

<220>
<223> Description of Artificial Sequence:primer for rat
      Nedd9 and Cas-L

<400> 3
tgactggagg gctctttg                                               18


<210> 4
<211> 20
<212> DNA
<213> Artificial Sequence

<220>
<223> Description of Artificial Sequence:primer for

beta-actin

<400> 4
ctatgagctg cctgacggtc                                                    20

<210> 5
<211> 833
<212> PRT
<213> Mus musculus

<400> 5
Met Trp Ala Arg Asn Leu Met Ala Arg Ala Leu Tyr Asp Asn Val Pro
1               5                   10                  15

Glu Cys Ala Glu Glu Leu Ala Phe Arg Lys Gly Asp Ile Leu Thr Val
            20                  25                  30

Ile Glu Gln Asn Thr Gly Gly Leu Glu Gly Trp Trp Leu Cys Ser Leu
        35                  40                  45

His Gly Arg Gln Gly Ile Val Pro Gly Asn Arg Val Lys Leu Leu Ile
    50                  55                  60

Gly Pro Val Gln Glu Thr Pro Gly His Glu Gln Pro Thr Pro Gly Pro
65                  70                  75                  80

Met His Gln Thr Phe Gly Gln Gln Lys Leu Tyr Gln Val Pro Asn Ser
                85                  90                  95

Gln Ala Ala Ser Arg Asp Thr Ile Tyr Gln Val Pro Pro Ser Tyr Gln
            100                 105                 110

Asn Gln Gly Ile Tyr Gln Val Pro Thr Gly His Gly Thr Gln Asn Gln
        115                 120                 125

Asp Val Tyr Gln Val Pro Pro Ser Phe Gln Arg Asn Ile Gly Gly Thr
    130                 135                 140

Asn Gly Pro Leu Leu Ser Lys Lys Val Ile Thr Pro Val Arg Thr Gly
145                 150                 155                 160

Leu Gly Tyr Val Tyr Glu Tyr Pro Ser Arg Tyr Gln Lys Asp Val Tyr
                165                 170                 175

Asp Val Pro Pro Ser His Ser Thr Gln Gly Val Tyr Asp Ile Pro Pro
            180                 185                 190

Ser Ser Val Lys Gly Pro Val Phe Ser Val Pro Val Gly Glu Ile Lys
        195                 200                 205

Pro Gln Gly Val Tyr Asp Ile Pro Pro Thr Gln Gly Val Tyr Ala Ile
    210                 215                 220

Pro Pro Ser Ala Cys Arg Asp Glu Ala Gly Leu Arg Glu Lys Glu Tyr
225                 230                 235                 240

Asp Phe Pro Pro Pro Met Lys Gln Asp Gly Lys Pro Asp Thr Arg Pro
                245                 250                 255

Glu Gly Val Tyr Asp Ile Pro Pro Thr Ser Thr Lys Thr Ala Gly Lys
            260                 265                 270

Asp Leu His Ile Lys Phe Pro Cys Asp Ala Pro Gly Gly Val Glu Pro
        275                 280                 285

Met Ala Arg Arg His Gln Ser Phe Ser Leu His His Ala Pro Ser Gln
    290                 295                 300

Leu Gly Gln Ser Gly Asp Thr Gln Ser Asp Ala Tyr Asp Val Pro Arg
305                 310                 315                 320

Gly Val Gln Phe Leu Glu Val Pro Thr Glu Thr Ser Glu Lys Ala Asn
                325                 330                 335

Pro Glu Glu Arg Asp Gly Val Tyr Asp Val Pro Leu His Asn Pro Ala
            340                 345                 350

Asp Ala Lys Gly Ser Arg Asp Val Val Asp Gly Ile Asn Arg Leu Ser
        355                 360                 365

Phe Ser Ser Thr Gly Ser Thr Arg Ser Asn Met Ser Thr Ser Ser Thr
    370                 375                 380

Ser Ser Lys Glu Ser Ser Leu Ser Ala Ser Pro Ser Gln Asp Lys Arg
385                 390                 395                 400

Leu Arg Leu Asp Pro Asp Thr Ala Ile Glu Lys Leu Tyr Arg Leu Gln
                405                 410                 415

Gln Thr Leu Glu Met Gly Val Cys Ser Leu Met Ser Leu Val Thr Thr
            420                 425                 430

Asp Trp Arg Cys Tyr Gly Tyr Met Glu Arg His Ile Asn Glu Ile Arg
        435                 440                 445

Thr Ala Val Asp Lys Val Glu Leu Phe Leu Arg Glu Tyr Leu His Phe
    450                 455                 460

Ala Lys Gly Ala Leu Ala Asn Ala Ser Cys Leu Pro Glu Leu Val Leu
465                 470                 475                 480

His Asn Lys Met Ser Arg Glu Leu Gln Arg Val Glu Asp Ser His Gln
                485                 490                 495

Ile Leu Ser Gln Thr Ser His Glu Leu Asp Glu Cys Ser Trp Ser Leu
            500                 505                 510

Asn Ile Leu Ala Ile Asn Lys Pro Gln Asn Lys Cys Asp Gly Leu Asp
        515                 520                 525

Arg Phe Val Met Val Ala Lys Thr Val Pro Asp Asp Ala Lys Gln Leu
    530                 535                 540

Thr Thr Thr Ile Ser Thr Tyr Ala Glu Thr Leu Phe Arg Ala Asp Pro
545                 550                 555                 560

Ala Asn Ser His Leu Lys Asn Gly Pro Asn Ser Ile Met Asn Ser Ser
                565                 570                 575

Glu Tyr Thr His Pro Gly Ser Gln Met Gln Pro Leu His Pro Gly Asp
            580                 585                 590

Tyr Lys Ala Gln Val His Ser Lys Pro Leu Pro Pro Ser Leu Ser Lys
        595                 600                 605

Asp Gln Pro Pro Asp Cys Gly Ser Ser Asp Gly Ser Glu Arg Ser Trp
    610                 615                 620

Met Asp Asp Tyr Asp Tyr Val His Leu Gln Gly Lys Glu Glu Phe Glu
625                 630                 635                 640

Arg Gln Gln Lys Glu Leu Leu Glu Lys Ala Asn Ile Met Lys Gln Ser
                645                 650                 655

Lys Ala Gln Leu Glu His His Gln Leu Ser Gln Phe Gln Leu Leu Glu

            660                 665                 670

Gln Glu Ile Thr Lys Pro Val Glu Asn Asp Ile Ser Lys Trp Lys Pro
        675                 680                 685

Ser Gln Ser Leu Pro Thr Thr Asn Asn Ser Val Gly Ala Gln Asp Arg
    690                 695                 700

Gln Leu Leu Cys Phe Tyr Tyr Asp Gln Cys Glu Thr His Phe Ile Ser
705                 710                 715                 720

Leu Leu Asn Ala Ile Asp Ala Leu Phe Ser Cys Val Thr Pro Ala Gln
                725                 730                 735

Pro Pro Arg Ile Phe Val Ala His Ser Lys Phe Val Ile Leu Ser Ala
            740                 745                 750

His Lys Leu Val Phe Ile Gly Asp Thr Leu Thr Arg Gln Val Ala Ala
        755                 760                 765

Gln Asp Ile Arg Asn Lys Val Arg Asn Ser Ser Asn Gln Leu Cys Glu
    770                 775                 780

Gln Leu Lys Thr Ile Val Met Ala Thr Lys Met Ala Ala Leu His Tyr
785                 790                 795                 800

Pro Ser Thr Thr Ala Leu Gln Glu Met Val His Gln Val Thr Asp Leu
                805                 810                 815

Ser Arg Asn Ala Gln Leu Phe Lys Arg Ser Leu Leu Glu Met Ala Thr
            820                 825                 830

Phe

<210> 6
<211> 833
<212> PRT
<213> Rattus norvegicus

<400> 6
Met Trp Ala Arg Asn Leu Met Ala Arg Ala Leu Tyr Asp Asn Val Pro
1               5                   10                  15

Glu Cys Ala Glu Glu Leu Ala Phe Arg Lys Gly Asp Ile Leu Thr Val
            20                  25                  30

Ile Glu Gln Asn Thr Gly Gly Leu Glu Gly Trp Trp Leu Cys Ser Leu
        35                  40                  45

His Gly Arg Gln Gly Ile Val Pro Gly Asn Arg Val Lys Leu Leu Ile
    50                  55                  60

Gly Pro Val Gln Glu Pro Ser Ser His Glu Gln Pro Thr Pro Gly Pro
65                  70                  75                  80

Met His Gln Asn Phe Gly Gln Gln Lys Leu Tyr Gln Val Pro Asn Ser
                85                  90                  95

Gln Ala Ala Pro Arg Asp Thr Ile Tyr Gln Val Pro Pro Ser Tyr Gln
            100                 105                 110

Asn Gln Gly Ile Tyr Gln Val Pro Thr Gly His Gly Thr Pro Glu Gln
        115                 120                 125

Asp Val Tyr Gln Val Pro Pro Ser Val Gln Arg Asn Ile Gly Ser Thr
    130                 135                 140

Asn Gly Pro His Leu Gly Lys Lys Val Ile Thr Pro Val Arg Val Gly
145                 150                 155                 160

His Gly Tyr Val Tyr Glu His Pro Ser Arg Tyr Gln Lys Asp Val Tyr
                165                 170                 175

Asp Val Pro Pro Ser His Thr Thr Gln Gly Val Tyr Asp Ile Pro Pro
            180                 185                 190

Ser Ser Val Lys Gly Pro Val Phe Ser Val Pro Val Gly Glu Leu Lys
        195                 200                 205

Pro Gln Gly Val Tyr Asp Ile Pro Pro Thr Gln Gly Val Tyr Ala Ile
    210                 215                 220

Pro Pro Ser Ala Cys Arg Asp Glu Ala Gly Leu Arg Glu Lys Glu Tyr
225                 230                 235                 240

Asp Phe Pro Pro Pro Met Lys Gln Asp Gly Lys Leu Asp Thr Arg Pro
                245                 250                 255

Glu Gly Val Tyr Asp Ile Pro Pro Thr Ser Thr Lys Thr Val Gly Lys
            260                 265                 270

Asp Leu His Thr Lys Phe Pro Cys Asp Ala Pro Gly Gly Ala Glu Pro
        275                 280                 285

Met Ala Arg Arg His Gln Ser Leu Ser Leu His His Ala Pro Ser Gln
    290                 295                 300

Val Gly Pro Ser Gly Asp Thr Gln Ser Asp Ala Tyr Asp Val Pro Arg
305                 310                 315                 320

Gly Val Gln Phe Leu Glu Val Pro Thr Glu Ser Ser Glu Lys Ala Asn
                325                 330                 335

Leu Glu Glu Arg Asp Gly Val Tyr Asp Val Pro Leu His Asn Pro Ala
            340                 345                 350

Asp Ala Lys Gly Ser Arg Asp Val Val Asp Gly Ile Asn Arg Leu Ser
        355                 360                 365

Phe Ser Ser Thr Gly Ser Thr Arg Ser Asn Met Ser Thr Ser Ser Thr
    370                 375                 380

Ser Ser Lys Glu Ser Ser Leu Ser Ala Ser Pro Ser Gln Asp Lys Arg
385                 390                 395                 400

Leu Leu Leu Asp Pro Asp Thr Ala Ile Gly Lys Leu Tyr Trp Leu Gln
                405                 410                 415

Gln Thr Leu Glu Thr Gly Ile Cys Ser Leu Met Ser Leu Val Thr Ile
            420                 425                 430

Asp Trp Arg Ser Tyr Gly Tyr Met Glu Arg His Ile Asn Glu Ile Arg
        435                 440                 445

Thr Ala Val Asp Lys Val Glu Leu Phe Leu Arg Glu Tyr Leu His Phe
    450                 455                 460

Ala Arg Gly Ala Leu Ala Asn Ala Ser Cys Leu Pro Glu Pro Val Leu
465                 470                 475                 480

Leu Asn Lys Met Asn Arg Glu Leu Gln Arg Val Asp Asp Ser His Gln
                485                 490                 495

Ile Leu Ser Gln Thr Ser His Asp Leu Asn Glu Cys Ser Trp Ser Leu
            500                 505                 510

Asn Val Leu Ala Ile Asn Lys Pro Gln Asn Lys Cys Asp Asp Leu Asp
        515                 520                 525

Arg Phe Val Met Ala Val Lys Thr Val Pro Asp Asp Ala Lys Gln Leu
    530                 535                 540

Thr Thr Thr Ile Ser Thr Tyr Ala Glu Thr Leu Phe Arg Val Asp Pro
545                 550                 555                 560

Gly Asn Ser His Leu Lys Asn Gly Pro Asp Ser Ile Thr Asn Ser Ile
                565                 570                 575

Glu Tyr Thr His Thr Gly Ser Gln Met Gln Pro Leu Arg Pro Gly Asp
            580                 585                 590

Tyr Lys Gly Gln Val His Asn Lys Pro Leu Pro Pro Thr Leu Ser Lys
        595                 600                 605

Glu Gln Pro Pro Asp Cys Ser Ser Ser Asp Gly Ser Glu Arg Ser Trp
    610                 615                 620

Met Asp Asp Tyr Asp Tyr Val His Leu Gln Gly Lys Glu Glu Phe Glu
625                 630                 635                 640

Arg Gln Gln Lys Glu Leu Leu Glu Lys Glu Asn Ile Met Lys Gln Ser
                645                 650                 655

Lys Ala Gln Leu Glu His His Gln Leu Ser Gln Phe Gln Leu Leu Glu
            660                 665                 670

Gln Glu Ile Thr Lys Pro Val Glu Asn Asp Ile Ser Lys Trp Lys Pro
        675                 680                 685

Ser Gln Ser Leu Pro Thr Thr Asn Asn Ser Val Gly Ala Gln Asp Arg
    690                 695                 700

Gln Leu Leu Cys Phe Tyr Tyr Asp Gln Cys Glu Thr His Phe Ile Ser
705                 710                 715                 720

Leu Leu Asn Ala Ile Asp Ala Leu Phe Ser Cys Val Ser Ser Ala Gln
                725                 730                 735

Pro Pro Arg Ile Phe Val Ala His Ser Lys Phe Val Ile Leu Ser Ala
            740                 745                 750

His Lys Leu Val Phe Ile Gly Asp Thr Leu Thr Arg Gln Val Ala Ala
        755                 760                 765

Gln Asp Ile Arg Asn Lys Val Arg Asn Ser Ser Asn Gln Leu Cys Glu
    770                 775                 780

Gln Leu Lys Thr Ile Val Met Ala Thr Lys Met Ala Ala Leu His Tyr
785                 790                 795                 800

Pro Ser Thr Thr Ala Leu Gln Glu Met Val His Gln Val Thr Asp Leu
                805                 810                 815

Ser Arg Asn Ala Gln Leu Phe Lys Arg Ser Leu Leu Glu Met Ala Thr
            820                 825                 830

Phe


<210>  7
<211>  833
<212>  PRT
<213>  Rattus norvegicus

<400> 7
Met Lys Tyr Lys Asn Leu Met Ala Arg Ala Leu Tyr Asp Asn Val Pro
1               5                   10                  15

Glu Cys Ala Glu Glu Leu Ala Phe Arg Lys Gly Asp Ile Leu Thr Val
            20                  25                  30

Ile Glu Gln Asn Thr Gly Gly Leu Glu Gly Trp Trp Leu Cys Ser Leu
        35                  40                  45

His Gly Arg Gln Gly Ile Val Pro Gly Asn Arg Val Lys Leu Leu Ile
    50                  55                  60

Gly Pro Val Gln Glu Pro Ser Ser His Glu Gln Pro Thr Pro Gly Pro
65                  70                  75                  80

Met His Gln Asn Phe Gly Gln Gln Lys Leu Tyr Gln Val Pro Asn Ser
                85                  90                  95

Gln Ala Ala Pro Arg Asp Thr Ile Tyr Gln Val Pro Pro Ser Tyr Gln
            100                 105                 110

Asn Gln Gly Ile Tyr Gln Val Pro Thr Gly His Gly Thr Pro Glu Gln
        115                 120                 125

Asp Val Tyr Gln Val Pro Pro Ser Val Gln Arg Asn Ile Gly Ser Thr
    130                 135                 140

Asn Gly Pro His Leu Gly Lys Lys Val Ile Thr Pro Val Arg Val Gly
145                 150                 155                 160

His Gly Tyr Val Tyr Glu His Pro Ser Arg Tyr Gln Lys Asp Val Tyr
                165                 170                 175

Asp Val Pro Pro Ser His Thr Thr Gln Gly Val Tyr Asp Ile Pro Pro
            180                 185                 190

Ser Ser Val Lys Gly Pro Val Phe Ser Val Pro Val Gly Glu Leu Lys
        195                 200                 205

Pro Gln Gly Val Tyr Asp Ile Pro Pro Thr Gln Gly Val Tyr Ala Ile
    210                 215                 220

Pro Pro Ser Ala Cys Arg Asp Glu Ala Gly Leu Arg Glu Lys Glu Tyr
225                 230                 235                 240

Asp Phe Pro Pro Pro Met Lys Gln Asp Gly Lys Leu Asp Thr Arg Pro
                245                 250                 255

Glu Gly Val Tyr Asp Ile Pro Pro Thr Ser Thr Lys Thr Val Gly Lys
            260                 265                 270

Asp Leu His Thr Lys Phe Pro Cys Asp Ala Pro Gly Gly Ala Glu Pro
        275                 280                 285

Met Ala Arg Arg His Gln Ser Leu Ser Leu His His Ala Pro Ser Gln
    290                 295                 300

Val Gly Pro Ser Gly Asp Thr Gln Ser Asp Ala Tyr Asp Val Pro Arg
305                 310                 315                 320

Gly Val Gln Phe Leu Glu Val Pro Thr Glu Ser Ser Glu Lys Ala Asn
                325                 330                 335

Leu Glu Glu Arg Asp Gly Val Tyr Asp Val Pro Leu His Asn Pro Ala
            340                 345                 350

Asp Ala Lys Gly Ser Arg Asp Val Val Asp Gly Ile Asn Arg Leu Ser
        355                 360                 365

Phe Ser Ser Thr Gly Ser Thr Arg Ser Asn Met Ser Thr Ser Ser Thr
    370                 375                 380

Ser Ser Lys Glu Ser Ser Leu Ser Ala Ser Pro Ser Gln Asp Lys Arg
385                 390                 395                 400

Leu Leu Leu Asp Pro Asp Thr Ala Ile Gly Lys Leu Tyr Trp Leu Gln
                405                 410                 415

Gln Thr Leu Glu Thr Gly Ile Cys Ser Leu Met Ser Leu Val Thr Ile
            420                 425                 430

Asp Trp Arg Ser Tyr Gly Tyr Met Glu Arg His Ile Asn Glu Ile Arg
        435                 440                 445

Thr Ala Val Asp Lys Val Glu Leu Phe Leu Arg Glu Tyr Leu His Phe
    450                 455                 460

Ala Arg Gly Ala Leu Ala Asn Ala Ser Cys Leu Pro Glu Pro Val Leu
465                 470                 475                 480

Leu Asn Lys Met Asn Arg Glu Leu Gln Arg Val Asp Asp Ser His Gln
                485                 490                 495

Ile Leu Ser Gln Thr Ser His Asp Leu Asn Glu Cys Ser Trp Ser Leu
            500                 505                 510

Asn Val Leu Ala Ile Asn Lys Pro Gln Asn Lys Cys Asp Asp Leu Asp
        515                 520                 525

Arg Phe Val Met Val Ala Lys Thr Val Pro Asp Asp Ala Lys Gln Leu
    530                 535                 540

Thr Thr Thr Ile Ser Thr Tyr Ala Glu Thr Leu Phe Arg Val Asp Pro
545                 550                 555                 560

Gly Asn Ser His Leu Lys Asn Gly Pro Asp Ser Ile Thr Asn Ser Ile
                565                 570                 575

Glu Tyr Thr His Thr Gly Ser Gln Met Gln Pro Leu Arg Pro Gly Asp
            580                 585                 590

Tyr Lys Gly Gln Val His Asn Lys Pro Leu Pro Pro Thr Leu Ser Lys
        595                 600                 605

Glu Gln Pro Pro Asp Cys Ser Ser Ser Asp Gly Ser Glu Arg Ser Trp
    610                 615                 620

Met Asp Asp Tyr Asp Tyr Val His Leu Gln Gly Lys Glu Glu Phe Glu
625                 630                 635                 640

Arg Gln Gln Lys Glu Leu Leu Glu Lys Glu Asn Ile Met Lys Gln Ser
                645                 650                 655

Lys Ala Gln Leu Glu His His Gln Leu Ser Gln Phe Gln Leu Leu Glu
            660                 665                 670

Gln Glu Ile Thr Lys Pro Val Glu Asn Asp Ile Ser Lys Trp Lys Pro
        675                 680                 685

Ser Gln Ser Leu Pro Thr Thr Asn Asn Ser Val Gly Ala Gln Asp Arg
    690                 695                 700

Gln Leu Leu Cys Phe Tyr Tyr Asp Gln Cys Glu Thr His Phe Ile Ser
705                 710                 715                 720

Leu Leu Asn Ala Ile Asp Ala Leu Phe Ser Cys Val Ser Ser Ala Gln
                725                 730                 735

Pro Pro Arg Ile Phe Val Ala His Ser Lys Phe Val Ile Leu Ser Ala
            740                 745                 750

His Lys Leu Val Phe Ile Gly Asp Thr Leu Thr Arg Gln Val Ala Ala
        755                 760                 765

Gln Asp Ile Arg Asn Lys Val Arg Asn Ser Ser Asn Gln Leu Cys Glu
    770                 775                 780

Gln Leu Lys Thr Ile Val Met Ala Thr Lys Met Ala Ala Leu His Tyr
785                 790                 795                 800

Pro Ser Thr Thr Ala Leu Gln Glu Met Val His Gln Val Thr Asp Leu
                805                 810                 815

Ser Arg Asn Ala Gln Leu Phe Lys Arg Ser Leu Leu Glu Met Ala Thr
            820                 825                 830

Phe


<210>  8
<211>  832
<212>  PRT
<213>  Mus musculus

<400>  8
Met Lys Tyr Lys Asn Leu Met Ala Arg Ala Leu Tyr Asp Asn Val Pro
  1               5                  10                  15

Glu Cys Ala Glu Glu Leu Ala Phe Arg Lys Gly Asp Ile Leu Thr Val
             20                  25                  30

Ile Glu Gln Asn Thr Gly Gly Leu Glu Gly Trp Trp Leu Arg Ser Leu
         35                  40                  45

His Gly Arg Gln Gly Ile Val Pro Gly Asn Arg Val Lys Leu Leu Ile
     50                  55                  60

Gly Pro Val Gln Glu Thr Pro Gly His Glu Gln Pro Thr Pro Gly Pro
 65                  70                  75                  80

Met His Gln Thr Phe Gly Gln Gln Lys Leu Tyr Gln Val Pro Asn Ser
                 85                  90                  95

Gln Ala Ala Ser Arg Asp Thr Ile Tyr Gln Val Pro Pro Ser Tyr Gln
            100                 105                 110

Asn Gln Gly Ile Tyr Gln Val Pro Thr Gly His Gly Thr Pro Glu Gln
        115                 120                 125

Asp Val Tyr Gln Val Pro Pro Ser Val Gln Arg Asn Ile Gly Gly Thr
    130                 135                 140

Asn Gly Pro Leu Leu Ser Lys Lys Val Ile Thr Pro Val Arg Thr Gly
145                 150                 155                 160

His Gly Tyr Val Tyr Glu Tyr Pro Ser Arg Tyr Gln Lys Asp Val Tyr
                165                 170                 175

Asp Val Pro Pro Ser His Ser Thr Gln Gly Val Tyr Asp Ile Pro Pro
            180                 185                 190

Ser Ser Val Lys Gly Pro Val Phe Ser Val Pro Val Gly Glu Ile Lys

        195                 200                 205

Pro Gln Gly Val Tyr Asp Ile Pro Pro Thr Gln Gly Val Tyr Ala Ile
    210                 215                 220

Pro Pro Ser Ala Cys Arg Asp Glu Ala Gly Leu Arg Glu Lys Glu Tyr
225                 230                 235                 240

Asp Phe Pro Pro Pro Met Lys Gln Asp Gly Lys Pro Asp Thr Arg Pro
                245                 250                 255

Glu Gly Val Tyr Asp Ile Pro Pro Thr Ser Thr Lys Thr Ala Gly Lys
            260                 265                 270

Asp Leu His Ile Lys Phe Pro Cys Asp Ala Pro Gly Gly Val Glu Pro
        275                 280                 285

Met Ala Arg Arg His Gln Ser Phe Ser Leu His His Ala Pro Ser Gln
    290                 295                 300

Leu Gly Gln Ser Gly Asp Thr Gln Ser Asp Ala Tyr Asp Val Pro Arg
305                 310                 315                 320

Gly Val Gln Phe Leu Glu Val Pro Thr Glu Thr Ser Glu Lys Ala Asn
                325                 330                 335

Pro Glu Glu Arg Asp Gly Val Tyr Asp Val Pro Leu His Asn Pro Ala
            340                 345                 350

Asp Ala Lys Gly Ser Arg Asp Val Val Asp Gly Ile Asn Arg Leu Ser
        355                 360                 365

Phe Ser Ser Thr Gly Ser Thr Arg Ser Asn Met Ser Thr Ser Ser Thr
    370                 375                 380

Ser Ser Lys Glu Ser Ser Leu Ser Ala Ser Pro Ser Gln Asp Lys Arg
385                 390                 395                 400

Leu Arg Leu Asp Pro Asp Thr Ala Ile Glu Lys Leu Tyr Arg Leu Gln
                405                 410                 415

Gln Thr Leu Glu Met Gly Val Cys Ser Leu Met Ser Leu Val Thr Thr
            420                 425                 430

Asp Trp Arg Cys Tyr Gly Tyr Met Glu Arg His Ile Asn Glu Ile Arg
        435                 440                 445

Thr Ala Val Asp Lys Val Glu Leu Phe Leu Arg Glu Tyr Leu His Phe
    450                 455                 460

Ala Lys Gly Ala Leu Ala Asn Ala Ser Cys Leu Pro Glu Leu Val Leu
465                 470                 475                 480

His Asn Lys Met Lys Arg Glu Leu Gln Arg Val Glu Asp Ser His Gln
                485                 490                 495

Ile Leu Ser Gln Thr Ser His Asp Leu Asn Glu Cys Ser Trp Ser Leu
            500                 505                 510

Asn Ile Leu Ala Ile Asn Lys Pro Gln Asn Lys Cys Asp Asp Leu Asp
        515                 520                 525

Arg Phe Val Met Val Ala Lys Thr Val Pro Asp Asp Ala Lys Gln Leu
    530                 535                 540

Thr Thr Thr Ile Ser Thr Tyr Ala Glu Thr Leu Phe Arg Ala Asp Pro
545                 550                 555                 560

Ala Asn Ser His Leu Lys Asn Gly Pro Asn Ser Ile Met Asn Ser Ser
                565                 570                 575

Glu Tyr Thr His Pro Gly Ser Gln Met Gln Pro Leu His Pro Gly Asp
            580                 585                 590

Tyr Lys Ala Gln Val His Ser Lys Pro Leu Pro Pro Ser Leu Ser Lys
        595                 600                 605

Asp Gln Pro Pro Asp Cys Gly Ser Ser Asp Gly Ser Glu Arg Ser Trp
    610                 615                 620

Met Asp Asp Tyr Asp Tyr Val His Leu Gln Gly Lys Glu Glu Phe Glu
625                 630                 635                 640

Arg Gln Gln Lys Glu Leu Leu Glu Lys Glu Asn Ile Met Lys Gln Ser
                645                 650                 655

Lys Ala Gln Leu Glu His His Gln Leu Ser Gln Phe Gln Leu Leu Glu
            660                 665                 670

Gln Glu Ile Thr Lys Pro Val Glu Asn Asp Ile Ser Lys Trp Lys Pro
        675                 680                 685

Ser Gln Ser Leu Pro Thr Thr Asn Asn Ser Val Gly Ala Gln Asp Arg
    690                 695                 700

Gln Leu Leu Cys Phe Tyr Tyr Asp Gln Cys Glu Thr His Phe Ile Ser
705                 710                 715                 720

Leu Leu Asn Ala Ile Asp Ala Leu Phe Ser Cys Val Ser Ser Ala Gln
                725                 730                 735

Pro Pro Arg Ile Phe Val Ala His Ser Lys Phe Val Ile Leu Ser Ala
            740                 745                 750

His Lys Leu Val Phe Ile Gly Asp Thr Leu Thr Arg Gln Val Ala Ala
        755                 760                 765

Gln Asp Ile Arg Asn Lys Val Arg Asn Ser Ser Asn Gln Leu Cys Glu
    770                 775                 780

Gln Leu Lys Thr Ile Val Met Ala Thr Lys Met Ala Ala Leu His Tyr
785                 790                 795                 800

Pro Ser Thr Thr Ala Leu Gln Glu Met Val His Gln Val Thr Asp Leu
                805                 810                 815

Ser Arg Asn Ala Gln Leu Phe Lys Arg Ser Leu Leu Glu Met Ala Thr
            820                 825                 830

## Description

### FIELD OF THE INVENTION

The present invention relates to pharmaceutical compositions comprising Nedd9 protein. The present invention also relates to pharmaceutical compositions comprising Nedd9 protein and FAK protein. Pharmaceutical compositions of the present invention can be used for the treatment of neuronal damages and neurodegenerative diseases, including ischemic injury such as transient cerebral global ischemia and cerebral stroke. Furthermore, the present invention relates to methods for differentiating neuronal cells.

### BACKGROUND OF THE INVENTION

The recent identification of neuronal stem cells in the adult mammalian brain suggests the possibility that the adult brain has a latent capacity for self-repair in response to ischemia. Ischemia is a powerful reformatting and reprogramming stimulus for the brain, which induces endogenous proteins related to the pathophysiology of the injured brain (Barone, F.C. and Feuerstein, G.Z. 1999. J. Cereb. Blood Flow Metab. 19:819-834). It has been shown that some pathological conditions such as ischemia induce neurogenesis in the adult mammalian brain (Liu, J. et al. 1998. J. Neurosci. 18:7768-7778). Recent findings have raised the possibility that the brain has as latent capacity for self-repair in response to injury or disease through the use of the endogenous NSCs or neural progenitor cells (Magavi, S.S. et al. 2000. Nature 405:951-955).

In focal ischemia model of mice, upregulation of several genes has been investigated and grouped as temporal episodes or "waves" of expression of different groups of genes (Barone, F.C. and Feuerstein, G.Z. 1999. J. Cereb. Blood Flow Metab. 19:819-834). Some of these proteins, such as Neurocan (Haas, C.A. et al. 1999. J. Neurosci. 19:9953-9963) and neuronal precursor cell-expressed, developmentally down-regulated gene (Nedd9)/Caspase2 (Kinoshita, M. et al. 1997. J. Cereb. Blood Flow Metab. 17:507-514), are primarily expressed by neurons or glia during embryonic development and strongly down-regulated during the early postnatal phase.

Nedd9 was initially identified as a neuronal precursor cell (NPC)-expressed, developmentally down-regulated gene in the mouse central nervous system (CNS) (Kumar, S. et al. 1992. Biochem. Biophys. Res. Commun. 185:1155-1161). Gene expression of Nedd9 is detected in embryonal day 10 and 14 and disappeared in adult mouse brain (Kumar, S. et al. 1992. supra). Later waves of new gene expression like Nedd9 include mediators that appear to be important in tissue remodeling and recovery of function (Read, S.J. et al. 2001. J. Cereb. Blood Flow Metab. 21:755-778), such as transforming growth factor-β (Wang, X. et al. 1995. Brain Res. Bull. 36:607-609) and osteopontin (Wang, X. et al. 1998. J. Neurosci. 18:2075-2083).

The product of Nedd9 was subsequently reported to be identical to the mouse Crk-associated substrate (Cas) lymphocyte-type (Cas-L) (Homology database available at the website of the National Center for Biotechnology Information located on the worldwide web at www.ncbi.nlm.nih.gov. Accessed November 4, 2003), which is also known as human enhancer filamentation 1 (HEF1) cloned by another group via a completely different strategy (Law, S.F. et al. 1996. Mol. Cell. Biol. 16:3327-3337). Human Cas-L was first identified in the present inventors' laboratory as a 105 kDa protein predominantly tyrosine phosphorylated by the ligation of β1 integrins in human leukemia H9 cells (Minegishi, M. et al. 1996. J. Exp. Med. 184:1365-1375). The major biological function of Cas-L is the restoration of IL-2 production by costimulation with β1 integrins and T-cell receptor (TCR) complex (Kamiguchi, K. et al. 1999. J. Immunol. 163:563-568), and the enhancement of cell migration by the engagement of β1 integrins and TCR complex, or β1 integrins alone (Ohashi, Y. et al. 1999. J. Immunol. 163:3727-3734). To exert these functions, it is necessary that Cas-L is associated with FAK or Pyk2 and is tyrosine phosphorylated by these kinases (Tachibana, K., et al. 1997. J. Biol. Chem. 272:29083-29090). The present inventors have demonstrated that Cas-L is a hematopoietic variant of p130Cas (Minegishi, M. et al., 1996. supra). P130Cas was identified as a 130-kDa protein that is highly tyrosine phosphorylated in v-Src- (Reynolds, A.B. et al. 1989. Mol. Cell. Biol. 9:3951-3958) and v-Crk-transformed cells (Sakai, R., et al., 1994. EMBO J. 13:3748-3756). These proteins and Efs/Sin compose the Cas family, which has a conserved amino acid secondary structure with numerous protein-protein interactions, such as Src-homology 3(SH3) domain, substrate domain, serine-rich domain, coiled-coil regions, helix-loop-helix domain, and C-terminal domain (Minegishi, M. et al., 1996. supra; O'Neill, G.M. et al. 2000. Trends in Cell Biology 10:111-119). These structures feature a docking molecule, which interacts with focal adhesion kinase (FAK) or proline-rich tyrosine kinase 2 (Pyk2). Although Nedd9/Cas-L is likely to play some roles in the development and differentiation of the CNS, its protein expression and function in fetal brain has not been reported. More importantly its function in relation to the pathogenesis of brain ischemia as well as the expression in adult brain remains unknown.

The nonreceptor tyrosine kinase FAK and Pyk2 were identified as potential common mediators of signaling by growth factors and integrins (Clark, E.A. and Brugge, J.S. 1995. Science. 268:233-239; Neet, K. and Hunter, T. 1996. Genes to Cells. 1:147-169; Avraham, H. et al. 2000. Cell. Signal. 12:123-133; Girault, J.A. et al. 1999. Trends Neurosci. 22:257-263). Pyk2 is highly expressed in the CNS (Menegon, A. et al. 1999. European Journal of Neuroscience. 11:3777-3788) and acts as an upstream regulator of MAPK signaling pathways in response to diverse cellular stimuli, including depolarization and increase of intracellular Ca²⁺ levels (Lev, S. et al. 1995. Nature. 376:737-745; Yu, H. et al. 1996. J. Biol. Chem. 271:29993-29998). Pyk2 is rapidly tyrosine-phosphorylated in cortical neurons and co-localized with activated p38MAPK in both neuron and microglia after focal cerebral ischemia in rats (Tian, D. et al. 2000. J. Neurosci. 20:6478-6487). FAK is also highly expressed in the CNS as a splice isoform FAK+6,7 mainly (Burgaya, F. et al. 1995. European Journal of Neuroscience. 7:1810-1821; Toutant, M. et al. 2000. Biochem. J. 348 Pt 1:119-128; Menegon, A. et al. 1999. supra). FAK has an important role in the prevention of apoptosis by cell attachment in non-neuronal cells (Frisch, S.M. et al. 1996. J. Cell Biol. 134:793-799; Hungerford, J.E. et al. 1996. J. Cell Biol. 135:1383-1390). Although FAK plays important roles in the development and differentiation of the CNS (Hungerford, J.E. et al. 1996. supra), there are few reports regarding its function in adult brain, especially in relation to the pathogenesis of brain ischemia.

Stroke is the second largest cause of death and the leading cause of long-term disability in Japan and United States. Much work has been devoted to decrease neuronal damage in order to reduce the resultant neurologic deficits of ischemia. Although most efforts for treating stroke aim at reducing or limiting the size of the ensuing acute lesion, this goal will not be accomplished for many patients, and treatment for later phase of stroke is unfortunately not well established. As the therapeutic time window toward acute hypoperfusion and energy failure is limited, identification of endogenous molecules involved in neuroprotection or remodeling of neurons would be potentially important for therapy.

### SUMMARY OF THE INVENTION

An objective of the present invention is to provide pharmaceutical compositions comprising Nedd9 protein. Another objective of this invention is to provide uses of Nedd9 protein for the manufacture of a medicament for treating neuronal damages or neurodegenerative diseases.

To identify an endogenous molecule involved in protection of nerves or remodeling of neurons, the present inventors cloned the mouse Cas-L gene and assessed the temporal profile of Nedd9/Cas-L, as well as its related molecules p130Cas, FAK, and PYK2, in brain of transient global ischemia in rats. The inventors also investigated its function in brain using mouse embryos, neurospheres, and transfectants of PC-12 cells. The results indicate that Nedd9 was a splicing variant of Cas-L and selectively induced in neurons of cerebral cortex and hippocampus 3 to 14 days after the ischemia. Induced Nedd9 protein was tyrosine phosphorylated and was bound to FAK in dendrite and soma of neurons after the ischemia. In physiological process, Nedd9 was transiently expressed in neurites and cell body of developing neurons in cerebral cortex and hippocampus during development. Nedd9 promoted neurite outgrowth of PC-12 cells in an NGF-independent manner. These results suggest that delayed upregulation of Nedd9 in neurons plays an important role in neuron remodeling after global ischemia in rats.

In one aspect, the present invention provides pharmaceutical compositions comprising Nedd9 protein. The pharmaceutical compositions may further comprise FAK protein.

In another aspect, the present invention provides uses of Nedd9 protein for the manufacture of a medicament for treating neuronal damages or neurodegenerative diseases, including ischemic injury such as transient global ischemia and stroke.

In still another aspect, the present invention provides methods for differentiating neuronal cells, the method comprising introducing a gene encoding Nedd9 protein into neuronal cells and allowing the expression of the gene in the cells. More specifically, the method comprises transforming neuronal cells with an expression vector comprising a gene encoding Nedd9 protein and incubating the cells under the conditions that allow the expression of the gene.

Nedd9 protein, which has been identified to an endogenous molecule involved in protection of nerves or remodeling of neurons, would be useful as a pharmaceutical agent for alleviating neuronal damages caused by ischemia such as stroke. Transplantation of Nedd9 positive neurons into the core of cerebral ischemia may help the recovery of brain tissue from ischemic insult, with such delivery system of neurons having been established recently (Borlongan, C.V. et al. 1998. Exp. Neurol. 149:310-321; Kondziolka, D. et al. 2000. Neurology 55:565-569). Furthermore, the fact that Nedd9 is not expressed in normal adult brain is useful for its application for therapy targeted to neuron, because its overexpression can facilitate remodeling of regenerated neurons specifically. As the range of targets for regeneration therapy has been widened, future study of Nedd9 expression may be applicable to other diseases such as neurodegenerative diseases (Alzheimer's, amyotrophic lateral sclerosis, cerebellar ataxias, Huntington's, Parkinson's, and progressive supranuclear palsy) or brain and spinal cord injury.

### BRIEF DESCRIPTION OF THE DRAWINGS

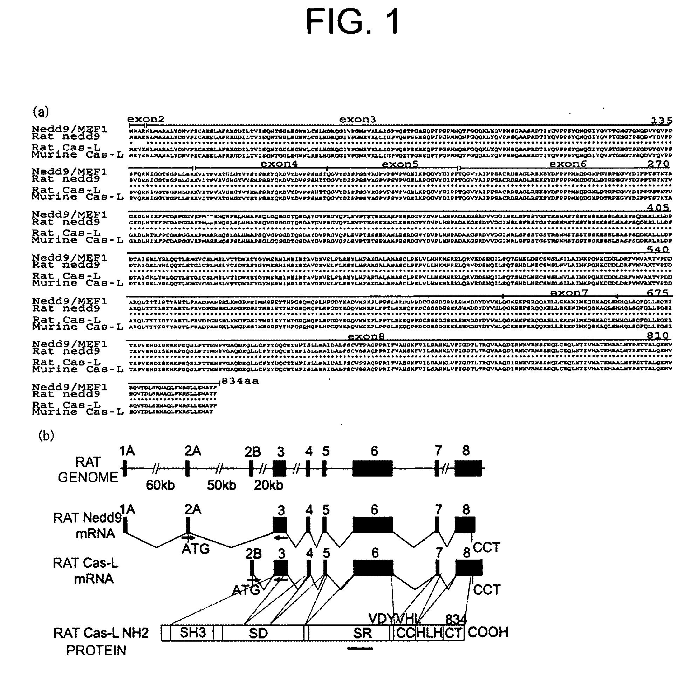

Fig. 1(a) shows comparison of the deduced amino acid sequences of Nedd9 and Cas-L from mouse and rat. Fig. 1(b) shows a proposed model for the generation of Nedd9 and Cas-L mRNA variants by multiple domain structure of Cas-L. (Top) The organization of the Nedd9/Cas-L gene. Exons are indicated by black boxes and numbered. (Middle) The presence of exons in the different mRNAs represented by boxes linked by connecting lines. Translation initiation sites in exons 2A and 2B and the translation stop site in exon 8 are indicated. Arrows indicate the primer sites for PCR amplification designed to give products from Nedd9 mRNA and Cas-L mRNA, respectively. (Bottom) The domain structure of Cas-L is demonstrated. A bar indicates the responsible domain to be recognized by anti-Cas-L antibody used in this study. SD: substrate domain, SR: serine-rich region, CC: coiled-coil regions, HLH: helix-loop-helix domain, CT: C-terminal domain.

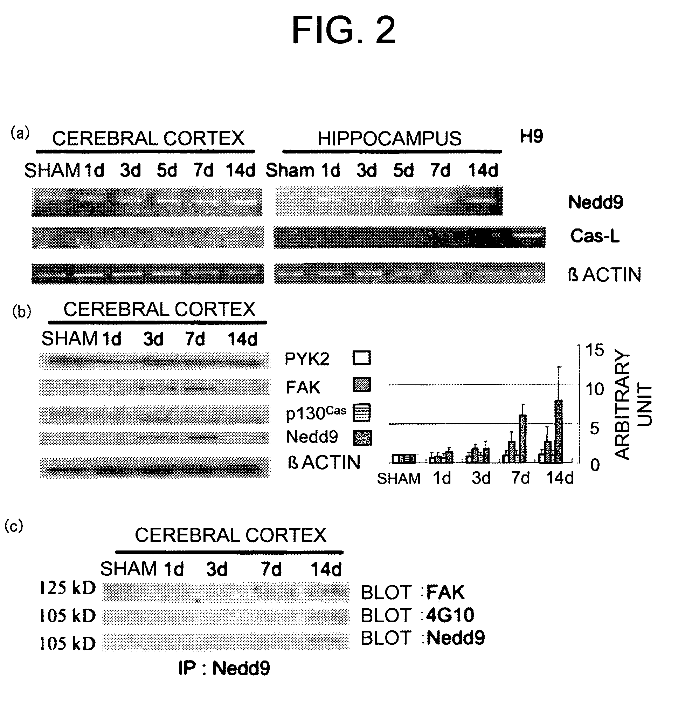

Fig. 2(a) shows a representative result of RT-PCR discriminating the two splicing variants of Nedd9 with β-actin used as an internal control. H9 cells serve as positive control of Cas-L. Fig. 2(b) presents photographs showing the expression of proteins related to Nedd9/Cas-L by western blot analysis. Both Nedd9 protein and FAK were up-regulated, consistent with the temporal profile of Nedd9 expression. However, other proteins did not exhibit significant changes. Fig. 2(c) presents photographs showing the FAK content in immunoprecipitates of Nedd9 at 0, 1, 3, 7 or 14 days following transient global ischemia. The FAK content in the immunoprecipitates was determined by visualization with the anti-FAK antibody. The immunoprecipitates were also blotted by anti-phosphotyrosine antibody 4G10.

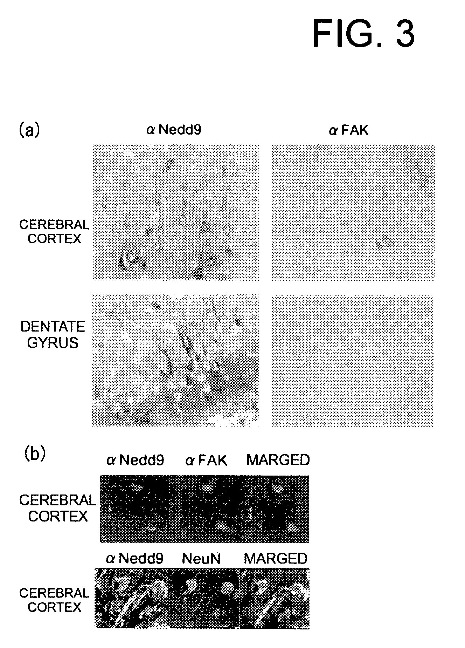

Fig. 3(a) presents photographs showing Nedd9-immunoreactive cells detected at the cerebral cortex and hippocampus. Rat cerebral cortex and hippocampal coronal slices reveal intense Nedd9-immunoreactivity in the neuronal dendrites. In contrast, the neuronal cell nucleus appeared devoid of Nedd9 staining. Fig. 3(b) presents photographs showing co-localization of Nedd9 and NeuN or FAK.

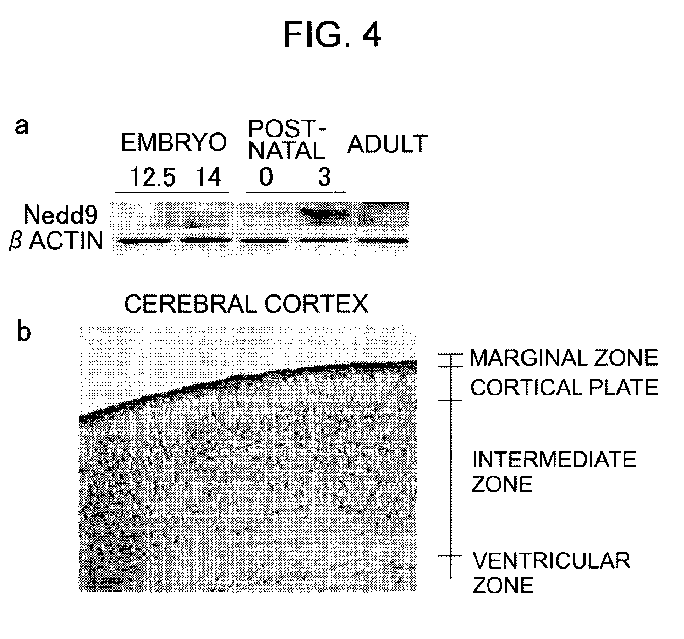

Fig. 4(a) presents photographs showing the results of Western blotting analysis of Nedd9 temporal expression during mouse brain development. Nedd9 expression was determined in developing brains at embryonal day 12.5 and 14, and postnatal day 0 and 3 in adult brain. Standard Western blot with anti-Nedd9 Ab showed expression of Nedd9 protein during natal and early postnatal phase of developing brains, and not in adult brains. Fig. 4 (b) presents a photograph showing immunohistochemical distribution of Nedd9 in the cerebral cortex and hippocampus at P0. Positive immunoreactivity with Nedd9 was fibrillary in the marginal zone and ventricular part of the cortical plate.

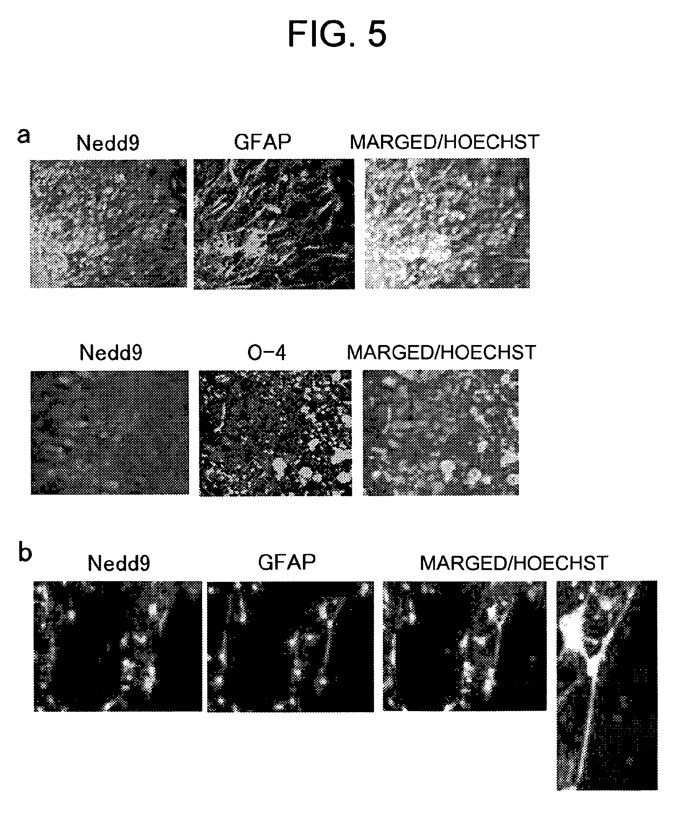

Fig. 5(a) presents photographs showing immunocytochemical distribution of Nedd9 (green), O-4/GFAP (red), and Hoechst 33258 (blue) in mouse neurosphere cells. Nedd9 was not expressed in oligodendroglia nor astroglia. Fig.5(b) presents photographs showing immunocytochemical distribution of Nedd9 (red), Tuj1 (green), and Hoechst33258 (blue) in mouse neurosphere cells. Nedd9 was expressed in developing neurons.

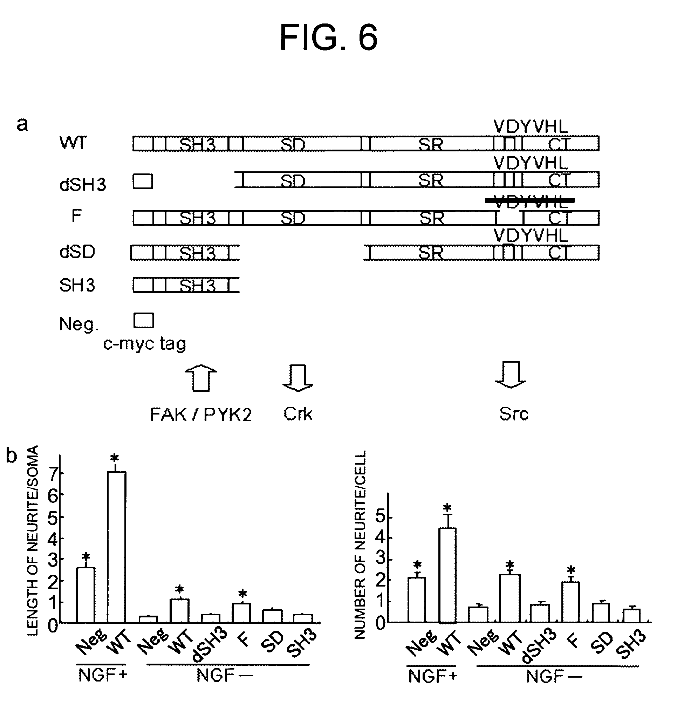

Fig. 6(a) schematically shows GFP-Nedd9 deletion mutants. Abbreviations: WT: wild-type, dSH3: delta SH3, SD: substrate domain. F: deletion in YDYVHL motif, SR: serine-rich domain, CC: coiled-coil regions, HLH: helix-loop-helix domain, CT: C-terminal domain. Fig. 6(b) shows neurite formation in the absence or presence of NGF. Data are expressed as neurite length averaged over diameter of soma (bar diagrams; y axis = cell diameters) and as number of neurites per cell. Statistical significance is tested with respect to unstimulated control PC-12 cells (*, p<0.001).

### DETAILED DESCRIPTION OF THE INVENTION

The present invention provides a pharmaceutical composition containing a Nedd9 protein as an active ingredient. A Nedd9 protein contained in a pharmaceutical composition of the present invention needs to promote neurite elongation and to contribute differentiation of nerve cells. Its origin and structure are not particularly limited. For example, it is possible to use Nedd9 proteins derived from humans, mice, rats, rabbits, dogs, horses, cats, pigs, cattle, goats, sheep, primates such as monkeys and gorillas, and other mammals. Nedd9 genes of desired species are prepared by utilizing techniques such as hybridization, PCR and the like using a probe or primer prepared based on nucleotide sequences encoding known Nedd9 proteins. Nedd9 proteins of various animals are thus prepared and can be used in this invention. In a pharmaceutical composition of the present invention, it is preferred to use a protein derived from the same kind of animals as a target for administration. In other words, it is preferred to use a human Nedd9 protein when the composition is administered to humans. The amino acid sequences of Nedd9 proteins of mice and rats are shown in SEQ ID NOs: 5 and 6, respectively, and both in Fig. 1a. The mRNA encoding mouse Nedd9 protein is registered as GenBank Accession No. NM 017464. Using the nucleotide sequence of the mRNA or a part thereof as a probe, a gene library or the like is screened to obtain Nedd9 mRNA of various mammals including humans. Then, various Nedd9 proteins of various mammals are prepared and can be used in the present invention.

In the present invention, the term "Nedd9 protein" includes wild type Nedd9 proteins as well as Nedd9 proteins having the same functions as the wild type protein and that are recombinantly or synthetically prepared, and then used.

Examples of Nedd9 proteins having the same functions as those of wild type Nedd9 proteins include proteins having high amino acid sequence homology with wild type Nedd9 proteins. The term "high amino acid sequence homology" means identity of 70% or more, preferably 80% or more, more preferably 90% or more, still more preferably 95% or more, furthermore preferably 97% or more, for example, 99%. The term "% identity" is defined as a function of the number of identical positions shared by two amino acid sequences, except any conservative substitution, when the two sequences are aligned with or without allowing gaps for optimal comparison purposes. The percent identity of amino acid sequences is a value obtained by dividing the number of identical residues by the total number of amino acid residues in the alignment. The identity of amino acid sequences can be calculated using a software known in the art, such as BLAST, BLAST-2, Megalign and the like. The regulable parameters necessary for calculation can be properly determined by persons skilled in the art, considering sensitivity or the like.

Proteins having high homology with such natural Nedd9 proteins can be prepared by, for example, the following manner. Using a polynucleotide encoding a known Nedd9 protein or a part thereof as a probe or primer, a gene encoding a target protein is isolated from a tissue expressing the gene or an appropriate gene library by a hybridization or PCR procedure and the isolated gene is expressed to prepare the protein (referred to Sambrook, J. et al. 1989 "Molecular Cloning: A Laboratory Manual, 2nd ed.", Vol. 1-3, Cold Spring Harbor Laboratory Press).

A Nedd9 protein contained in a pharmaceutical composition according to the present invention may be a variant of a known Nedd9 protein produced by deleting a part of the protein or by adding amino acid residue(s), or another polypeptide, wherein the variant protein has the same function as wild type Nedd9. Methods for deletion, insertion, replacement, addition and modification of any amino acid residue in a protein having a known amino acid sequence are well known. In accordance with the known methods, a Nedd9 protein contained in a pharmaceutical composition according to the present invention can also be modified for any purposes such as for increasing the in vivo activity, for stabilization or the like. Preferable modified Nedd9 proteins in the present invention are conservatively modified proteins having the same biological activity as the natural Nedd9 protein. For example, one or more amino acid residues can be modified by glycosylation or phosphorylation. Proteins can be modified by chemical or enzymatic de-glycosylation or de-phosphorylation. Proteins may also be modified by adding one or more amino acid residues to the N terminal or the C terminal of a natural amino acid sequence, or deleting one or more amino acid residues. Moreover, proteins may be modified by inserting one or more amino acid residues into any portion of a natural amino acid sequence. Alternatively, proteins can be modified by replacing one or more amino acid residues in a natural amino acid sequence with another amino acid residue(s). Such replacement is carried out by substitution with amino acid residue(s) having the same side chain as the amino acid residue to be substituted. This is called conservative amino acid substitution. In the conservative amino acid substitution, mutually exchangeable amino acids are classified in accordance with the chemical properties of their side chains into the following groups:
(1) Neutral hydrophobic side chain (alanine, valine, leucine, proline, tryptophan, phenyl alanine or methionine),
(2) Neutral polar side chain (glycine, serine, threonine, tyrosine, cysteine, asparagines or glutamine),
(3) Basic side chain (lysine, arginine or histidine),
(4) Acidic side chain (aspartic acid or glutamic acid),
(5) Aliphatic side chain (glycine, alanine, valine, leucine or isoleucine),
(6) Aliphatic hydroxyl group side chain (serine or threonine),
(7) Amine-containing side chain (asparagines, glutamine, lysine, arginine or histidine),
(8) Aromatic side chain (phenyl alanine, tyrosine or tryptophan), and
(9) sulfur-containing side chain (cysteine or methionine).

The neurite growth accelerating activity of a modified Nedd9 protein is measured to confirm whether the biological activity is retained, thereby determining whether it can be used as a pharmaceutical composition of the present invention. The retention of the biological activity does not always mean the same activity level as that of natural proteins. It is enough to have the same activity as the activity of natural proteins. The activity level may be higher than or somewhat lower than the level of natural proteins.

The present invention indicates that Nedd9 protein and FAK protein are simultaneously expressed in vivo and it is considered that they collaboratively act on differentiation to neurons in the developing brain.

Therefore, the present invention also relates to a pharmaceutical composition containing a FAK protein in addition to a Nedd9 protein. Similar to the case of Nedd9 protein, the origin and structure of a FAK protein contained in a pharmaceutical composition of the present invention are not particularly limited as long as the protein binds to a Nedd9 protein and promotes elongation of neurites. In a pharmaceutical composition of the present invention, it is preferred to contain a protein derived from the same kind of animals as a target for administration (when administered to humans, a human FAK protein is preferably used). Preferable examples of FAK proteins include proteins having an amino acid sequence encoded by mRNA registered as GenBank Accession No. L13616 (Whitney, G.S. Chan, P.Y. Blake, J. Cosand, W.L. Neubauer, M.G. Aruffo, A. and Kanner, S.B. 1993, "Human T and B lymphocytes express a structurally conserved focal adhesion kinase, pp125FAK." DNA Cell Biol, 12(9): 823-30). Similar to the case of the Nedd9 protein, in accordance with necessity, a modified FAK is prepared by modifying a natural amino acid sequence, and the resulting modified FAK may be contained in a pharmaceutical composition of the present invention.

A method for preparing recombinant proteins is well known. Nedd9 protein and FAK protein can be prepared using an appropriate expression system including in vitro system and host-vector system. For example, an expression vector including a chimera gene composed of an appropriate transcription/translation regulatory region and a protein coding sequence is constructed. The term "transcription/translation regulatory region" means a DNA sequence necessary for expressing an operably linked protein coding sequence in a specific host cell, and includes a promoter, an enhancer, a polyadenylation signal, an operator sequence, a ribosome-binding site, an initiation signal, and the like. For expression in a eukaryotic cell, a promoter/enhancer element is preferably contained in an expression vector to control the desired protein expression in host cells. The term "operably" linked or bound means that a protein coding sequence connected downstream a control sequence is expressed in a host by the direction of the control sequence without displacement of the reading frame. Examples the expression vector include known various vectors suitable for expression of mammalian cells, bacteria cells, insect cells, plant cells, yeast cells, and the like. These vectors may be used for the production of Nedd9 protein and FAK protein. Host cells are transformed with the constructed expression vector. Various cells are established as host cell lines. Persons skilled in the art could readily select a method of introducing an expression vector suitable for the selected host cell. Prokaryotic cells can be transformed by, for example, calcium treatment or electroporation. Plant cells can be transformed by, without limitation, a known method of using agrobacterium. For mammalian cells, a calcium phosphate precipitation method can be exemplified. Additionally, there are known methods such as nuclear microinjection, protoplast fusion, DEAE-dextran method, cell fusion, electric pulse perforation method, lipofectamine method (GIBCO BRL) and a method of using FuGENE6 reagent (Boehringer-Mannheim). The transformation of mammalian cells can refer to, for example, the descriptions in Keown, W.A. et al. Methods in Enzymol, 1990, 185:527-37 and Mansour, S.L. et al. Nature, 1988, 336: 348-52. To secure the glycosylation of natural proteins, it is desired to use mammalian cells as a host cell. Examples of mammalian cells include known various cells such as CHO, COS, 293, A431, 205, CV-1, Hela, BHK, H1-60, U937, Hak and Jurkat cells. These cells can be used for the preparation of Nedd9 protein and FAK protein.

A host cell in which a desired gene has been introduced is cultured, a desired protein is expressed, and the protein is recovered from the resulting culture. It is also possible to obtain a desired protein from a culture medium in such a way that a secretory signal sequence recognized in a host is added to the desired protein and thereby the desired protein is secreted extracellularly. If necessary, a transgenic animal is prepared, and Nedd9 protein and FAK protein used in the present invention may be prepared from the animal. When the protein production is carried out with a transgenic animal, the protein is preferably expressed using a promoter capable of tissue-specific expression (for example, protein expression in a milk of a mammal).

In a pharmaceutical composition of the present invention, a protein is preferably contained as a main ingredient in a physiologically and pharmaceutically acceptable carrier. Nedd9 protein and/or FAK protein, which are main ingredients, are desirably contained in a substantially purified state in the carrier. The term "substantially purified" means the state not containing contaminants in the environment, namely, the state not containing proteins that coexist with Nedd9 protein or FAK protein in nature or proteins derived from hosts, which may be contaminated during the preparation of recombinant proteins. Substantially purified proteins can be prepared by chromatography such as gel filtration, reversed phase, adsorption, ion exchange and hydrophobic interaction, and other purification methods using known procedures such as ethanol precipitation, electrophoresis, evaporation, solvent extraction, immunoprecipitation, dialysis, re-crystallization, and ammonium sulfate precipitation. Protein purification may also be carried out by affinity chromatography utilizing a polyclonal or monoclonal antibody capable of specifically recognizing a protein to be purified. However, a pharmaceutical composition of the present invention may contain, as an active ingredient, proteins other than Nedd9 protein and FAK protein. That is, a pharmaceutical composition of the present invention may optionally contain active ingredients other than Nedd9 protein or FAK protein. Preferable active ingredients optionally added are ingredients that do not inhibit the activities of Nedd9 protein and FAK protein, and can treat or prevent nerve disorders or neurodegerative diseases by action mechanism different from these proteins. Example of the active ingredients include members of family such as fibloblast growth factors (FGF) (basic FGF; acidic FGF; FGF-3; FGF-4; FGF-5; FGF-6, FGF-7; FGF-8 and FGF-9), neurotrophines(NT) (nerve growth factor (NGF); brain derived nerve neutrient factor (BDNF); NT-3; NT4/5 and NT-6), insulin like growth factor (IGF) (IGF-1 and IGF-2), ciliary neutrophic factor, epidermal growth factors (EGF) (EGF; TGF α; heparin binding EGF; amphiregulin; beta-cellulin; vaccinia growth factor and neu differentiation factor), transforming growth factors β(TGF β), platelet derived growth factor, and vascular endothelial growth factor (VEGF). Other examples include leukocyte inhibitory factor (LIF), oncostatine, interleukin-11(IL-11), IL-6, and IL-1. Furthermore, in addition to the above proteins, a pharmaceutical composition of the present invention may contain compounds that do not inhibit the activities of Nedd9 protein and FAK protein, and that can treat or prevent nerve disorders or neurodegerative diseases by action mechanism different from these proteins. For example, compounds that bind to Tacrolimus binding proteins (FKBP) such as FK-506 reportedly stimulate the growth of a neurite in a nerve cell (Lyons, W.E. et al. Proc. Natl. Acad. Sci. USA. 1994, 91:3191-5 U.S. Patent 5,696,135). When the growth of neuroglial cells, neuriglial astrocyte cells and the like around the part where a pharmaceutical composition of the present invention is administered is undesirable, it is possible to use known antimitotic agents such as cytosine, arabinoside, 5-fluorouracil, hydroxyurea, methotrexate, and the like.

A pharmaceutical composition of the present invention is preferably formulated into an injection. For example, a pharmaceutical composition of the present invention may be administered in a cerebral ventricle or injected to an affected area.

Further, Nedd9 protein and/or FAK protein may be bound to an agent that confers desired pharmaceutical or pharmacological properties. For example, it is possible to prepare a pharmaceutical composition of the present invention so that the administered proteins can be delivered to a target affected site through blood-brain barrier and to administer the composition by intravenous injection. Furthermore, it is also possible to selectively deliver the proteins into the target cells by incorporating Nedd9 protein and/or FAK protein in a carrier such as a liposome or a polymer whose surface is bound to a molecule capable of selectively binding to a specific cell surface molecule such as antibody, ligand, receptor or the like.

The term "carrier" used herein means a substance capable of dispersing and retaining proteins having catalytic functions. Any substance can be used as the carrier as long as, when used at doses and concentrations for administration, it has no toxicity to target animals and does not inhibit the functions of the proteins that are main ingredients. Examples of the carrier include buffer solutions of phosphate, citrate, or other organic acid salts, glucose solution, and sterile water. Nedd9 protein and/or FAK protein dissolved in such a solution can be lyophilized if necessary and kept in a germ-free ampoule. The lyophilized product can be reconstituted with sterile water just prior to administration and then administered. Furthermore, it is also possible to incorporate Nedd9 protein and/or FAK protein in a solid, semi-solid or compatible substrate to be embedded in a tissue in need of treatment.

If necessary, a pharmaceutical composition of the present invention may be combined with additional ingredients such as an excipient, a diluent, a lubricant, a humectant, an emulsifier, a suspending agent, a preservative, a sweetener, an aromatic, a flavoring agent, a stabilizer, an antioxidant, a surfactant, a disintegrating agent, a binder, a viscosity stabilizer, and a permeating agent. In order to control the releasing rate of the active ingredients after the administration, the composition can be prepared as an appropriate dosage form using known methods. Poly(lactic acid-co-glycolic acid) (PLGA) polymer is known as a sustained-release agent.

A pharmaceutical composition of the present invention can be used for treating nerve disorders or neurodegenerative diseases. The "treatment" used herein includes treatment for therapy and treatment for prophyraxis. A pharmaceutical composition of the present invention can be administered to patients in need of treatment. The patients in need of treatment include patients suffering from a disease and persons who do not have a disease yet but are suspected to have a disease. Nerve disorders or neurodegenerative diseases to be treated by a pharmaceutical composition of the present invention is preferably damages and diseases in the brain or the spinal cord. Examples of nerve disorders include ischemic diseases such as transient global cerebral ischemia or cerebral stroke. Examples of neurodegenerative diseases include Alzheimer's disease, amyotrophic lateral sclerosis, cerebellar ataxias, spinal damyotrophy, Huntington's disease, Parkinson's disease, and progressive supranuclear palsy.

A pharmaceutical composition of the present invention can be used for mammals as a target. The target is not limited to mammals, but include humans, domesticated animals such as pets, and farm animals such as livestock. More specifically, any animals classified in mammals such as humans, dogs, cats, horses, cattle, sheep, mice, rats and the like, are exemplified. A pharmaceutical composition of the present invention is preferably used to treat humans.

The dose of a pharmaceutical composition of the present invention should be sufficient to induce the final differentiation of neurons, and varies depending on the dosage form, administration method, and patient's symptom, weight, sex, and age. The daily dose to an adult (weighing 60 kg) ranges usually from 0.1 µg to 10 g, preferably 1 µg to 1 g, more preferably 100 µg to 100 mg. For patients other than humans, the composition containing proteins in an amount calculated based on weight may be administered.

The present inventors confirmed that Nedd9 promotes neurite outgrowth of PC-12 cell when overexpressed in the cells. Therefore, the present invention relates to a method of differentiating a neuronal cell, the method comprising transforming a cell with an expression vector comprising a gene encoding a Nedd9 protein, culturing the cell under conditions that the gene is expressed. An expression vector used in this method comprises an appropriate transcription/translation regulatory region, which is recognized by a cell to be transformed, and a gene encoding a Nedd9 protein, which is operably linked to the transcription/translation regulatory region. The expression vector may further contain a marker for selecting the cell transformed with the vector. The markers are not particularly limited and includes those capable of detecting transformants based on thymidinekinase activity, antibiotic resistance, phenotype of transformants or the presence or absence of formation of inclusion bodies by a baculovirus. Cells to be transformed may be any cells capable of differentiating to neurons, and include immature neurons at any developmental stage. Such cells include neural stem cells and hematopoietic stem cells and can be isolated from bone marrow, peripheral blood, cord blood and the like. The isolation and cloning of such stem cell lines from brains of rodents and humans have been already succeeded (Snyder, E.Y. et al. Proc. Natl. Acad. Sci. USA. 1997. 94:11663-8; Gage, F.H. et al. Proc. Natl. Acad. Sci. USA. 1995. 92: 11879-83; Kuhn, H.G. et al. J. Neurosci. 1997. 17: 5820-9; U.S. Patents 5,270,191, 5,750,376, 5,753,506, 5,968,829).

In the culturing step of the method for differentiation of neuron cells according to the present invention, for example, cells can be cultured in a medium such as DMEM supplemented with serum. The culture is maintained in an appropriate culture vessel under conditions of 5% CO₂ and 37°C. The culturing is preferably carried out with sufficient aeration and shaking. Culturing conditions used in the method of the present invention are not limited to the above conditions, and any conditions are used as long as the cell culture is maintained and the differentiation to neurons can occur. The culturing is preferably carried out at pH and temperature near to those in physiological conditions. For example, the culturing is carried out at pH of from 6 to 8, preferably at near pH 7 (e.g. pH 7.4) at a temperature of from 30 to 40°C, preferably 32 to 38°C, more preferably 35 to 37°C. Nerve growth factors and/or nerve stimulants necessary for the growth and differentiation to neurons may be added to the medium. Examples thereof may include members of family such as FGF (basic FGF; acidic FGF; FGF-3; FGF-4; FGF-5; FGF-6; FGF-7; FGF-8 and FGF-9), NT (NGF; BDNF; NT-3; NT4/5; NT6), IGF (IGF-1; IGF-2), ciliary neutrophic growth factors, EGF (EGF; TGFα; heparin binding EGF; amphiregulin; beta-cellulin; vaccinia growth factor and neu differentiation factor), TGF β, platelet-derived growth factors, VEGF and the like, LIF, oncostatine M, IL-11, and IL-6.

Terminally differentiated neurons prepared by the method of the present invention can be used in transplantation for the treatment of various nerve disorders or neurodegerative diseases. When used in transplantation, it is preferred that stem cells be taken from a patient and, if needed, cultured or concentrated in vitro prior to transplantation. A desired neuronal cell can be selected based on the presence of a marker protein on the cell surface (for example, microtubule-associated protein 2 (MAP2); tau; β-tubulin; neurofilament L or M; nerve-specific enolase; and NeuN). For cell transplantation, the neuronal cells can be administered directly in the spinal fluid of a stroke cavity (cerebral intraventricular, intrathecal or intracisternal), or the periphery of an affected area. The cells are preferably administered in a state contained in a physiologically acceptable liquid carrier. The cell administration site can be determined by, for example, standard CT and MRI scan. The cells can be administered in an amount of generally 10⁶ to 10¹² cells, preferably 10⁷ to 10¹¹ cells, and more preferably 10⁸ to 10¹⁰ cells in total. The cell administration may be carried out at once or, if necessary, plural times at proper intervals.

The present invention reveals that overexpression of Nedd9 protein and FAK protein would be effective particularly for treatment of cerebral ischemia. Therefore, nervus disorders and neurodegerative diseases such as cerebral ischemia can be treated by introducing, in place of Nedd9 protein and FAK protein, genes encoding these proteins so that the genes are expressed in an affected area or its periphery. Gene tranfer may be carried out by an in vivo method of directly introducing genes in the body or an ex vivo method of introducing a gene into a cell taken from a patient and thereafter returning the cell in the body. The in vivo method is preferred because it is simple and easy.

The above gene transfer can be carried out in accordance with a known gene therapy method using virus vectors or non-virus vectors. Examples of known virus vectors include retrovirus, adenovirus, adeno-related virus, vaccinia virus, poxvirus, Sendai virus, herpes virus, poliovirus, immunodeficiency virus and other vectors derived from various DNA or RNA viruses. As a non-virus method for introducing a desired gene, known techniques include those utilizing liposome, polylysine complex, artificial viral envelope or beads such as colloidal gold particles. Gene therapy methods can be referred to, for example, "Supplement experimental medicine, Basic techniques of gene therapy" (Yodosha 1996), "Supplement experimental medicine, Experimental methods for gene transfer and expression analysis" (Yodosha 1997) and "Gene therapy development and research handbook" edited by The Japan Society of Gene Therapy (NTS; 1999). Another treatment can also be possible by obtaining a Nedd9 positive neuron and implanting the neuron in an affected area or its periphery.

The present invention will be explained in detail below with reference to examples, but it is not to be construed as being limited thereto.

### EXAMPLE

### 1. Experimental Methods

### (1) Antibodies and Reagents

Monoclonal antibodies against pp125FAK, p130Cas, and PYK2 were obtained from Transduction Laboratories (Lexington, KY). Anti-phosphotyrosine antibody (4G10) was purchased from Upstate Biotechnology, Inc. (Lake Placid, NY). Anti-β actin polyclonal antibody (pAb) (Santa Cruz Biotechnology, Santa Cruz, CA), mouse anti-neuron specific nuclear protein (NeuN, Chemicon), normal rabbit serum (Sigma, St. Louis, MO), normal mouse IgG1 (Sigma), and goat anti-mouse and goat anti-rabbit conjugated to HRP (Promega, Madison, WI) were purchased. Production of pAb specific to Cas-L has been described previously (Ohashi, Y. et al. 1998. J. Biol. Chem. 273:6446-6451; Miyake-Nishijima, R. et al. 2003. Arthritis Rheum. 48:1890-1900). Briefly, rabbits were immunized with glutathione S-transferase (GST) fusion protein of Cas-L (amino acids 419-524). Those portions of each protein were selected because of the relatively low homology of amino acid sequences with p130Cas. After the sera of anti-Cas-L and anti-p130Cas were absorbed with GST-p130Cas, specific antibodies for Cas-L pAb was affinity purified with GST-Cas-L. Basically, immunoprecipitates with anti-Cas-L pAb do not contain p130Cas (Ohashi, Y. et al. 1998. J. Biol. Chem. 273:6446-6451). Affinity-purified rabbit anti-mouse antibody was purchased from Jackson Laboratories (West Grove, PA). All other chemicals were purchased from Sigma Chemical Co. (St. Louis, MO) unless otherwise stated. Protein A Sepharose beads were from Pharmacia Biotech (Uppsala, Sweden).

### (2) cDNA cloning and (automated cycle) sequencing of murine Cas-L/HEF1

A λgt1 human placenta cDNA library (Clontech) was screened by hybridization with a 32P-labeled probe for 16 h at 50°C in a solution of 50 mM Tris-HCl (pH 7.5), 1 M NaCl, 1% SDS, and 100 µg/ml sonicated salmon testis DNA, and then washed at 65°C in 0.1 x SSC (SSC; 150 mM NaCl, 15 mM tri-sodium citrate, pH 7.0) containing 1% SDS. Approximately 1.0 x 10⁶ plaques were spread on petri dishes and lifted onto nylon filters (colony/plaque screen, NEN Research Products, Boston, MA) for screening. The hybridization probe used was the full-length cDNA for human Cas-L/HEF1, which was labeled with [α-32P] dCTP (Amersham) by the random primer labeling method (Feinberg, A.P. and Vogelstein, B. 1983. Anal. Biochem. 132:6-13). The hybridization-positive clones were analyzed by restriction enzyme mapping and nucleotide sequencing, which was carried out via primer walking and sub-cloning. Sequencing of the pBluescript phagemids (Stratagene) subclones was performed by ABI Dyedeoxy Terminator Cycle Sequencing kit (Applied Biosystems, Foster City, CA) and analyzed on DNA sequencing systems (model 373A, Applied Biosystems). The open reading frame of murine Cas-L was sequenced completely on both strands using internal primers.

The present inventors are the first to demonstrate that Cas-L and Nedd9 are splicing variants. The genetic difference between Cas-L and Nedd9 is just three amino acids in the first portion of open reading frame (Fig. 1a).

### (3) Animals

For transient global ischemia, male Sprague-Dawley rats weighing 250-350 g were anesthetized by an intraperitoneal injection of pentobarbital sodium (40 mg/kg). The body temperature was continuously monitored and maintained at 37.0-37.5 °C during ischemia and reperfusion with a thermostatically controlled heating pad connected to a rectal thermistor probe. The unilateral femoral vein and a tail artery were catheterized for induction of exsanguination and continuous monitoring of the blood pressure, respectively. The blood pressure was recorded continuously using a multi-pen recorder via a pressure transducer. Both of the common carotid arteries were isolated and loops made of PE-10 polyethylene catheter were passed around the carotid arteries for subsequent occlusion.

Global ischemia was induced by occlusion of both common arteries using polyethylene loops with systemic hypotension. The mean arterial pressure was maintained below 50 mmHg by drawing blood from the femoral vein (Smith, M.L. et al. 1984. Acta Neurol. Scand. 69:385-401). The focal cerebral blood flow was measured by laser-Doppler flowmetry in several cases to ensure that ischemia had been induced. After 21 min of ischemia, releasing the polyethylene loops around the common carotid arteries and normalizing the blood pressure achieved cerebral reperfusion.

Physiological parameters (blood pressure, PaO₂, PaCO₂, and the pH of arterial blood) were measured before induction of ischemia.

For developmental study, ICR mice were purchased from Charles River Japan Inc. The day of birth was designated as embryonal day (E) 12.5, 14, and postnatal day (P) 0, 3.

### (4) Reverse transcription-polymerase chain reaction

The cerebral cortex and hippocampus were separated after administration of excess sodium pentobarbital, the brains of sham-operated rats and rats subjected to 1 to 14 days of reperfusion after the transient global ischemia were removed from the skull immediately. H9 T-cell lines were cultivated for positive control of Cas-L mRNA. The isolation of total RNA from the samples was immediately performed using ISOGEN agent (Nippon Gene Co. LTD., Tokyo, Japan). RNA content was measured with a spectrophotometer (DU7500, Beckman, USA). Complementary DNA (cDNA) was synthesized from the RNA samples and polymerase chain reaction (PCR) was performed using BcaBESTTM RNA PCR Kit (Takara Biomedicals, Tokyo, Japan). Briefly, first-strand cDNA aliquots were mixed with 2.5 mmol/L MgCl2, 0.5 mmol/L dNTP, 2.5 U / 100 µl Bca-Optimized Taq polymerase, and 0.2 µmol of the following primers: forward 5'-AAATGTGGGCGAGGAAT-3' (SEQ ID NO: 1) for rat Nedd9, 5'-AGGGCTCATCTGACCAC-3' (SEQ ID NO: 2) for rat Cas-L and reverse 5'-TGACTGGAGGGCTCTTG-3' (SEQ ID NO: 3) for both cDNA. PCR cycles were as follows; 95°C, 3 min: 94°C, 1 min: 55.6°C, 1 min: 72°C, 2 min (30 cycles): 72°C, 2 min. The cDNA of a house keeping gene, β-actin, was amplified as a control under identical conditions using the following primers: 5'-CTATGAGCTGCCTGACGGTC-3' (SEQ ID NO: 4).

The fact that only Nedd9 but not Cas-L expression was induced in response to ischemia recapitulated Nedd9 physiological role since it is expressed in NPCs during brain development (Fig. 2a). The difference in expression between Nedd9 and Cas-L in brain may be due to the transactivation of the Nedd9 gene mediated by Nuclear Factor-kappa B, heat shock factor 1 or other related transcription factors known to be induced gradually after ischemia, since their binding sites exist in the upstream region of the rat Nedd9 gene. The molecular mechanism of Nedd9 gene regulation in these neurons will be an important subject for further studies.

### (5) Immunoprecipitation and immunoblotting

Protein extraction from rats and mice was performed under anesthesia with pentobarbital 40 mg/kg and ether inhalation, respectively. Tissues of cerebral cortex and hippocampus of rats were cut into pieces 1, 3, 7, and 14 days after reperfusion and those of whole brain of fetal mouse E12.5, E 14, P0, P3 and adult mouse were also cut into pieces. They were homogenized gently by bouncing 30 times in a glass tissue grinder in 1 ml of cold suspension buffer (20 mmol/L HEPES-KOH[pH 7.5], 250 mmol/L sucrose, 10 mmol/L KCI, 1.5 mmol/L MgCl2, 1 mmol/L EDTA, 1 mmol/L EGTA, 1 mmol/L dithiothreitol, 0.1 mmol/L phenylmethylsulfonyl fluoride, 2 µg/mL aprotinin, 10 µg/mL leupeptin, and 5 µg/mL pepstatin). Protein concentrations were determined by the Comassee Method (Bio-Rad, Hercules, CA, U.S.A.). For immunoprecipitation, the lysate were immunoprecipitated with anti-Cas-L pAb and protein A Sepharose beads. The precipitated or tissue lysates were mixed with 1x or 3x sample buffer, respectively, and boiled at 95°C for 10 minutes. Aliquots containing appropriate amount of protein were separated by 8% SDS-PAGE and electrotransferred onto PVDF membranes (Millipore Corp., Bedford, Massachusetts, USA). After blocking with 5% skim milk, the membranes were blotted with the first Ab in TBS: anti-Cas-L pAb (1:1000), anti-PYK2 mAb (1:1000), anti-FAK mAb (1:1000), anti p130Cas mAb (1:1000). They were washed with TBS-T, incubated with HRP-conjugated anti-rabbit or anti-mouse IgG Ab in TBS-T. The membranes were developed by the enhanced chemiluminescence (ECL) system (Amersham Corp., Arlington Heights, IL).

### (6) Densitometric analysis

The amounts of proteins were determined by densitometry. They were relative to the amount of protein in the sham-operated rats. Each amount of protein was normalized to the amount of the protein loaded on the lane.

### (7) Histological examinations

For histological examinations for transient global ischemia, rats were deeply anesthetized at 0, 1, 3, 7, or 14 days after forebrain ischemia with excess pentobarbital sodium (100 mg/kg) and perfused transcardially with heparinized saline followed by 4% paraformaldehyde/PBS for tissue fixation. Brains were postfixed in 4% paraformaldehyde/PBS at 4°C and parafinized sections were made. The parafinized sections (10 µm) were dewaxed and washed in PBS three times. The sections were permeabilized with 0.1% Triton X-100/PBS at room temperature for 10 minutes and then blocked in 4% FBS/PBS for 60 minutes. Subsequently, they were incubated with primary antibodies at 4°C overnight. Primary Abs and their dilutions were as follows: anti-Cas-L rabbit pAb, 1:200; anti-FAK mAb, 1:200; NeuN, 1:200. Biotinylated anti-rabbit IgG and anti-rabbit IgG at 1:1000 was used as the secondary antibody.

For developmental study, pups and adults anesthetized by ether inhalation were perfused through the left ventricle with 4% paraformaldehyde in 0.1 M PBS, pH 7.4, and then the brains were dissected and post-fixed overnight at 4°C in the same fixative. Sections (8 µm) were cut on a microtome and mounted on MAS-coated slides. The visualization of Cas-L was carried out with tyramide signal amplification using TSATM Systems (FITC, NEL701, NEMTM Life Science Products, Boston, MA, U.S.A.).

### (8) Neurosphere Culture

The basic culture medium, containing 20 ng/ml epidermal growth factor (Sigma) and 10 ng/ml FGF2 (R & D Systems), and the procedures for the neurosphere formation and differentiation assays were as described (Nakamura, Y. et al. 2000. J. Neurosci. 20:283-293). Briefly, cells from the subventricular zone of the P0 striatum were used for primary sphere formation (1 × 10⁵ cells per ml). After 24 h of incubation, spheres were mechanically dissociated, and plated onto matrigel-coated coverslips. These slides were processed 1, 3, and 5 days later for triple-labeled indirect immunocytochemistry to detect three major cell types: neurons, oligodendrocytes, and astrocytes, using TuJ1, anti-04, and anti-GFAP antibodies, respectively. The phenotype of the progenitor was determined based on the cell types (neuron, astrocyte, and/or oligodendrocyte) that were present in the clone, regardless of the cell number. Approximately 24 spheres per each animal were examined in three independent experiments.

### (9) Gene transfer using retrovirus

Retroviral gene transfer was carried out using Ping-pong infection method. Briefly, Plat-E cells (Gojo, S. et al. 1996. Cell Transplantation. 5:S81-S84) were seeded at a density of 2 x 10⁵ cells/well in a 6-well plate. The next day, cells were transfected using the Fugene6 reagent (Boehringer Mannheim) with 1 µg of pMX-Cas-Lwt-IRES-GFP, pMX-SH3-IRES-GFP, pMX-Cas-LF-IRES-GFP, pMX-Cas-LdeltaSH3-IRES-GFP, pMX-Cas-LdeltaSD-IRES-GFP, or pMX-IRES-GFP. Forty-eight hours post-transfection, the viral supernatant was collected, filtered through a 0.45 mm polysulfone filter and used to infect PT-67 cells (Clontech). After establishing PT-67 infectants stably producing each retrovirus, the supernatant were collected and then used to infect PC-12 cells at 32°C with 5 µg/ml of polybrene (Sigma). 96 hours post-infection, the infected cells were placed in regular growth media.

### (10) Assessment of neurite outgrowth of PC-12 cells

Infected PC-12 cells were cultured in Dulbecco's modified Eeagle's medium plus 10% fetal calf serum and 5% horse serum. For neuritogenesis, 5 x 10⁴ cells were seeded per 35-mm plate, grown overnight, and starved (0.5% fetal calf serum plus 0.5% horse serum) for 16-20 h. To this medium was then added 50ng/ml NGF. After 4 days of stimulation, neurite length was measured on photographed fields containing 100-250 cells. Data were expressed in two ways; first, as neurite length averaged over diameter of soma (bar diagrams; y axis = cell diameters); second, as number of neurites per cell. The level of statistical significance was assessed by a two-tailed Students' t test (unequal variance). All key conclusions were reconfirmed on independent clones and/or pools of clones selected en masse.

### 2. Results

### (1) Cloning of mouse Cas-L revealed that Nedd9 and Cas-L were splicing variants but their functional domains were identical to each other.

In order to clone the mouse Cas-L cDNA, human Cas-L cDNA was used as a probe to screen a mouse placenta cDNA library of lambda phage. Using the cloned mouse Cas-L cDNA, sequence homology searches were performed with cDNA database and genomic database. Nedd9 was found to share high degrees of sequence homology with mouse Cas-L in base sequence and amino acid sequence (Fig. 1a). Analysis of mouse genomic database revealed that Nedd9 was a splicing variant of Cas-L, and functional domains of both proteins were identical to each other. The rat genomic database showed that the deduced amino acid sequence of mouse and rat Nedd9 were highly conserved between the species. Rat Nedd9 shared high degrees of sequence homology with rat Cas-L, both in base sequence and amino acid sequence (Fig. 1a, b).

Although the function of Nedd9 has not been investigated, it can be presumed from that of Cas-L in other cell types since the functional domains of Nedd9 are identical to those of Cas-L (Fig. 1b). According to current knowledge, the general signaling pathway for Cas-L can be proposed thusly: following integrin receptor ligation by means of binding to the extracellular matrix, the Cas-protein-associated kinase FAK/PYK2 undergoes autophosphorylation, creating a binding site for Crk and other proteins (O'Neill, G.M. et al. 2000. Trends in Cell Biology. 10:111-119). Cas-L binds to the C-terminal FAK/PYK2 poly-proline region via their SH3 domains (Minegishi, M.et al. 1996. J. Exp. Med. 184:1365-1375; Astier, A. et al. 1997. J. Biol. Chem. 272:19719-19724) and are tyrosine phosphorylated directly by FAK/PYK2 (Astier, A. et al. 1997. J. Biol. Chem. 272:19719-19724; Tachibana, K. et al. 1997. J. Biol. Chem. 272:29083-29090).

### (2) Nedd9, not Cas-L, was transcriptionally upregulated and tyrosine phosphorylated along with FAK in cerebral cortex and hippocampus after transient global ischemia.

**Table 1**

| shows physiological parameters just prior to the induction of ischemia. The values in Table 1 are expressed as means +/- S.E.M.). | | | |
|---|---|---|---|
| sacrifice day | pH | O₂(mmHg) | PPCO₂ (mmHg) |
| sham treatment | 7.415+/-0.022 | 73.1+/-2.2 | 35.8+/-2.9 |
| 1 day after ischemia | 7.445+/-0.011 | 84.7+/-3.8 | 38.3+/-3.0 |
| 3 days after ischemia | 7.442+/-0.003 | 77.0+/-3.1 | 39.0+/-1.9 |
| 5 days after ischemia | 7.409+/-0.014 | 84.9+/-2.4 | 38.9+/-2.6 |
| 7 days after ischemia | 7.437+/-0.023 | 90.7+/-4.7 | 37.9+/-4.6 |
| 14 days after ischemia | 7.449+/-0.019 | 84.8+/-6.1 | 40.2+/-1.8 |

There were no significant differences in the parameters among the groups, demonstrating that all rats used in the ischemia study had no significant difference in their condition. Expression of Nedd9 and Cas-L gene in rats with global ischemia was analyzed by RT-PCR using mRNA derived from the animals at 1, 3, 5, 7, or 14 d after 21-min ischemia or at 1 h after sham operation. The RT-PCR was performed using a set of primers that amplifies the fragments from rat Nedd9 mRNA and Cas-L mRNA (Fig. 1b). The expression of Nedd9 was upregulated in both cerebral cortex and hippocampus around 1 to 14 days after ischemia. In contrast, the expression of Cas-L was not observed (Fig. 2a).

In order to address the pathway of signal transudation related to the upregulation of Nedd9, the present inventors examined the expression of other proteins related to Cas-L/Nedd9, i.e., FAK, PYK2, p130cas, by Western blot analysis. PYK2 and FAK, non-receptor tyrosine kinase, are the most intensively studied interactive partners in their signal transductions (O'Neill, G.M. et al. 2000. Trends in Cell Biology. 10:111-119) and p130Cas is another member of the Cas protein family. Both Nedd9 protein and FAK were upregulated in the same time course of Nedd9 expression, whereas other proteins did not exhibit significant changes (Fig. 2b). To determine whether Nedd9 protein and FAK combined to be upregulated after transient global ischemia, Nedd9 was immunoprecipitated at 0, 1, 3, 7 or 14 days following transient global ischemia, and the FAK content of immunoprecipitates was determined by visualization with anti-FAK antibody. The immunoprecipitates were also blotted by the anti-phosphotyrosine antibody 4G10 since activated Cas-L/Nedd9 is known to be tyrosine phosphorylated. The data hence demonstrated that the Nedd9 protein appeared to bind to FAK and be tyrosine-phosphorylated to comparable level with similar kinetics as observed with Nedd9 protein accumulation.

In this study, expression of Nedd9 protein and FAK was simultaneously induced in the cerebral cortex and hippocampus 3 to 14 days after 21 min transient global ischemia (Fig. 2b). Induced Nedd9 protein was tyrosine phosphorylated and was also co-immunoprecipitated with FAK after the ischemia (Fig. 2c).

### (3) Nedd9 protein was localized in dendrite-like structure and soma of neurons in cerebral cortex and hippocampus.

To evaluate the cellular and subcellular distributions of Nedd9 and FAK, rat brain slices of 7 days after the ischemic insult were stained for Nedd9 and FAK. Nedd9 and FAK-immunoreactive cells were detected at the cerebral cortex and hippocampus (Fig. 3a). The rat cerebral cortex and hippocampal coronal slices revealed intense Nedd9 immunoreactivity in the dendrite-like structures and cytosols. In contrast, the nucleus appeared devoid of Nedd9 staining. Co-localization of Nedd9 protein and FAK or NeuN confirmed the fact that Nedd9 protein was expressed with FAK in neurons (Fig. 3b).

Results from histochemical analysis revealed that induced Nedd9 protein as well as FAK was localized in dendrite-like structure and soma of neurons in the cerebral cortex and hippocampus (Fig. 3a, b). Considering the results of histochemical analysis, previous reports of Cas-L that is now revealed as a splicing variant of Nedd9 in (1) above, and results that Nedd9 protein and FAK expression were induced by transient global ischemia in (2) above, the present inventors postulated that Nedd9 was required for neuronal differentiation, being primarily expressed in the embryonic brain but not in the fully differentiated adult neuron. In short, the upregulation of Nedd9 in adult brain appears to play a role in remodeling neurons after ischemia.

### (4) Nedd9 is highly expressed in neurites of neurons at the marginal zone and ventricular part of cortical plate of embryonic and early postnatal phase of mouse brain.

In order to clarify the physiological role of Nedd9, the present inventors examined its temporal expression during the embryonic and postnatal phases of mouse brains, since Nedd9 has been reported to be transcriptionally expressed during embryonic phase and disappeared in adult (Kumar, S. et al. 1992. Biochem. Biophys. Res. Commun. 185:1155-1161). Western blot analysis showed that Nedd9 protein was expressed during the embryonic and postnatal phases, peaking at P3, and not in adult (Fig. 4a). This result is consistent with that disclosed in the previous report (Kumar, S. et al. 1992. Biochem. Biophys. Res. Commun. 185:1155-1161).

Immunohistochemistry revealed that Nedd9 was expressed at the distal end of dendrite-like structure in neurons of marginal zone and ventricular part of cortical plate (Fig. 4b). This staining pattern suggested that Nedd9 was mostly localized to neurites of neurons undergoing the later phase of differentiation, but not completely differentiated neurons. Whereas the markers of progenitor cells, such as proneural bHLH factors, are expressed in ventricular zone and subsequently give rise to neurons and glia, expression of bHLH differentiation genes such as the NeuroD/Nex family begins in the immature neuron and is maintained during neuronal differentiation at the cortical plate (Schwab, M.H. et al. 1998. JNeurosci 18, 1408-1418). Thus, the expression pattern of Nedd9 suggested that Nedd9 mediated the terminal differentiation of neurons. In mammals, FAK is expressed at the highest level in brain and is enriched in neurons (Andr, E. and Becker-Andr, M. 1993. Biochem Biophys Res Commun 190, 140-147; Burgaya, F. et al. 1995. European Journal of Neuroscience. 7:1810-1821; Grant, S.G. et al. 1995. Genes Dev 9, 1909-1921). Burgaya et al reported that FAK is found in the developing brain and is considered to be important for neurite outgrowth and maintenance (Burgaya, F. et al. 1995. European Journal of Neuroscience. 7:1810-1821). FAK protein levels are maximal at the embryonic life and decreased progressively during the postnatal life (Xie, Z. et al. 2003. Cell 114, 469-482), consistent with the temporal expression of Nedd9. Thus, Nedd9 and FAK were co-expressed and may collaborate to differentiate neurons in the developing brain.

### (5) Nedd9 was expressed in differentiating neurons from neuronal progenitor cells.

To further elucidate the expression pattern of Nedd9 during neuronal differentiation, we prepared cultures of stem/progenitor cells from the neurogenic subventricular zone of newborn mouse brain. Clones derived from single cell suspensions were expanded and cultured as neurospheres under nonadherent conditions in the presence of EGF, bFGF and then were plated with 10% fetal bovine serum to trigger substrate attachment and differentiation. Cells as the centers of the cultured neurospheres proliferate as stem/progenitor cells, whereas those that migrate to the culture periphery differentiate into neurons, oligodendroglia, and astroglia. Co-localization study was performed with the neuronal, oligodendroglial, and astroglial markers O-4, GFAP, and Tuj1, respectively. Nedd9 was co-expressed with Tuj 1 but not with O-4 and GFAP, indicating that Nedd9 was expressed in developing neurons (Fig. 5a, b). Nedd9 expression was localized at proximal neurites and soma of neurons, while Tuj 1 was stained at distal part of neurites and soma (Fig. 5b).

In this study we confirmed that Nedd9 protein was expressed in Tuj 1 and positive cells in neurosphere (Fig. 5b). These findings suggest that Nedd9 is expressed in mouse neural progenitor cells, consistent with the results from our immunohistochemical analysis that Nedd9 protein was expressed in the marginal zone and ventricular part of the cortical plate in neurites of immature neurons of developing mouse brain. Coexpression of Nedd9 and Tuj 1 at the perinuclear region of neuronal progenitor cells revealed that Nedd9 plays a role in microtubule organization since Tuj 1 is a beta tubulin type III protein specific to neurons. Recently, Xie et al. have reported that FAK is also expressed at the perinuclear region of cortical cultured neurons. They demonstrated that Cdk5 phosphorylation of FAK is critical for neuronal migration through the regulation of a microtubule fork (Xie, Z. et al. 2003. Cell 114, 469-482). Cas-L, a Nedd9 homologue, localizes mainly to focal adhesions and proximal stress fibers and reorganizes the cytoskeleton in response to various external environmental triggers (O'Neill, G.M. et al. 2000. Trends in Cell Biology 10:111-119). These data suggest the involvement of Nedd9 in microtubule-mediated functions such as neural migration or neural differentiation.

### (6) Nedd9 promoted neuronal outgrowth of PC-12 cells in NGF-independent manner.

The expression of Nedd9 in neurons undergoing differentiation in mouse embryo and neurosphere studies led us to examine the effect of Nedd9 on neurite outgrowth in PC-12 cells. We subcloned myc-tagged Nedd9 and its mutants into the pMx-IRES-GFP vector and infected it into PC-12 cells (Fig. 6a). Four days later NGF or vehicle was added the infected cells and expression of GFP and Nedd9 was assessed for the appearance of neurites. In contrast to cells infected with empty vector and mutants such as delta SH3, SH3, and delta SD, those expressing exogenous wild type Nedd9 showed promotion of neurite genesis in the presence or absence of NGF (Fig. 6b). These findings suggested that a Nedd9-dependent function contributed to promote the differentiation of PC-12 cells in NGF-independent manner.

In the present study we demonstrated that Nedd9 had a great impact on NGF-independent neurite outgrowth when overexpressed in PC-12 cells. The fact that expression of delta SH3, SH3, and delta substrate domain inhibited neurite outgrowth is compatible with the function of components of Nedd9, since Nedd9/Cas-L binds to FAK via their SH3 domains (Minegishi, M. et al. 1996. J. Exp. Med. 184:1365-1375) and substrate domain is required for interaction with SH2-containing proteins, such as Crk, Nck, and SH-PTP2. Crk has been reported to induce differentiation of PC-12 in an NGF-independent manner (Tanaka, S. et al. 1993. Mol. Cell. Biol. 13:4409-4415). Altum-Gultekin et al reported that v-Crk-expressing PC-12 cells exhibit neurite outgrowth and upregulation of FAK expression (Altun-Gultekin, Z.F. et al. 1998. Mol. Cell. Biol. 18:3044-3058), supporting that FAK is involved in NGF-independent neurite-outgrowth of PC-12 cells. These results thus confirm the hypothesis that delayed expression of Nedd9 and FAK may contribute to remodeling of neurons after ischemic injury in brain.

Having now fully described the present invention in some detail by way of illustration and example for purposes of clarity of understanding, it will be obvious to one of ordinary skill in the art that the same can be performed by modifying or changing the invention within a wide and equivalent range of conditions, formulations and other parameters without affecting the scope of the invention or any specific embodiment thereof, and that such modifications or changes are intended to be encompassed within the scope of the appended claims.

All publications, patents and patent applications mentioned in this specification are indicative of the level of skill of those skilled in the art to which this invention pertains, and are herein incorporated by reference to the same extent as if each individual publication, patent or patent application was specifically and individually indicated to be incorporated by reference.

## Claims

1. A pharmaceutical composition comprising Nedd9 protein in a physiologically acceptable carrier.

2. The pharmaceutical composition of claim 1, further comprising FAK protein.

3. The pharmaceutical composition of claim 1, wherein said composition is for the treatment of neuronal damage or a neurodegenerative disease.

4. Use of Nedd9 protein for the manufacture of a medicament for treating neuronal damage or a neurodegenerative disease.

5. Use of Nedd9 protein and FAK protein for the manufacture of a medicament for treating neuronal damage or a neurodegenerative disease.

6. The use of claim 4 or 5, wherein said medicament is for treating ischemic injury.

7. The use of claim 6, wherein said ischemic injury is transient global ischemia or stroke.

8. The use of claim 4 or 5, wherein said neuronal damage or a neurodegenerative disease is in the brain or spinal cord.

9. The use of claim 4 or 5, wherein said treatment results in terminal differentiation of an immature neuronal cell to neuron.

10. The use of claim 4 or 5, wherein said treatment comprises transplantation of Nedd9 positive neurons into the core of cerebral cortex of said patient.

11. A method of differentiating an immature neuronal cell to neurons, wherein said method comprises transforming an immature neuronal cell with an expression vector comprising a gene encoding Nedd9 protein, and incubating the cell under the conditions that the gene is expressed.

12. The method of claim 11, further comprising a step of contacting the cell with NGF.

13. The method of claim 11, wherein Nedd9 protein is a sequence homologue of the Cas-L protein.

14. The method of claim 11, wherein Nedd9 protein has identical functional domains to that of the Cas-L protein.

15. A composition comprising neurons obtained by the method of claim 11.

16. A composition comprising Nedd9 positive neuron and a physiologically acceptable carrier.

17. The composition of claim 16, wherein said composition is for the treatment of neuronal damage or a neurodegenerative disease.

18. A composition for differentiating an immature neuronal cell to neurons, comprising Nedd9 protein.
